# EUROPEAN PATENT SPECIFICATION

(11) **EP 0 975 771 B1**
(45) Date of publication and mention of the grant of the patent: **11.07.2007**
(21) Application number: 98918247.2
(22) Date of filing: 16.04.1998
(51) Int. Cl.: C12N 15/62, C07K 14/71, A61K 38/17

(54) **TYPE II TGF-BETA RECEPTOR/IMMUNOGLOBULIN CONSTANT REGION FUSION PROTEINS**
TYP II TGF-BETA RECEPTOR/IMMUNOGLOBULIN KONSTANTE DOMÄNE FUSIONSPROTEINE
PROTEINES DE FUSION CONTENANT LE RECEPTEUR TYPE II DU TGF-BETA ET PROTEINES DE FUSION EN TANT QUE REGION CONSTANTE D'UNE IMMUNOGLOBULINE

(30) Priority: 18.04.1997 US 44641 P
(43) Date of publication of application: 02.02.2000
(73) Proprietor: Biogen Idec MA Inc., Cambridge, Massachusetts 02142 (US)
(72) Inventor: GOTWALS, Philip, West Roxbury, MA 02132 (US); KOTELIANSKY, Victor, Boston, MA 02215 (US); CATE, Richard, Weston, MA 02193 (US); SANICOLA-NADEL, Michelle, Winchester, MA 01890 (US)
(74) Representative: Vossius & Partner
(86) International application number: PCT/US1998/007587
(87) International publication number: WO 1998/048024

(56) References cited:
- WO-A-94/17828
- WO-A-96/26964
- OKADOME T ET AL: "Distinct roles of the intracellular domains of transforming growth factor-beta type I and type II receptors in signal transduction." J BIOL CHEM, DEC 9 1994, 269 (49) P30753-6, XP002077575 UNITED STATES
- ANDERS RA ET AL: "Chimeric granulocyte/macrophage colony-stimulating factor/transforming growth factor-beta (TGF-beta) receptors define a model system for investigating the role of homomeric and heteromeric receptors in TGF-beta signaling." J BIOL CHEM, SEP 6 1996, 271 (36) P21758-66, XP002077576 UNITED STATES
- ISAKA Y. ET AL: 'Gene therapy by TGF-beta-Receptor-IgG Fc chimera inhibited extracellular matrix accumulation in experimental glomerulonephritis' JOURNAL OF THE AMERICAN SOCIETY OF NEPHROLOGY vol. 7, no. 9, 1996, UNITED STATES, page 1735
- DATABASE UNIPROT [Online] 01 October 1994 LIN H.Y.: 'TGF-beta receptor type II precursor (EC 2.7.1.37) (TGFR-2) (TGF-beta type II receptor)'

### SEQUENCE LISTING

<110> BIOGEN, INC.
<120> Type II TGF-Beta Receptor Fusion Proteins and Methods
<130> A018PCT
<140> PCT/US98/07587
   <141> 1998-04-16
<150> 60/044,641
   <151> 1997-04-18
<160> 13
<170> PatentIn Ver. 2.0
<210> 1
   <211> 9077
   <212> DNA
   <213> Rabbit
<220>
   <223> Description of Unknown Organism: Rabbit
<400> 1
   gaattcgagc ttgcatgcct gcaggtcgtt acataactta cggtaaatgg cccgcctggc 60
   tgaccgccca acgacccccg cccattgacg tcaataatga cgtatgttcc catagtaacg 120
   ccaataggga ctttccattg acgtcaatgg gtggagtatt tacggtaaac tgcccacttg 180
   gcagtacatc aagtgtatca tatgccaagt acgcccccta ttgacgtcaa tgacggtaaa 240
   tggcccgcct ggcattatgc ccagtacatg accttatggg actttcctac ttggcagtac 300
   atctacgtat tagtcatcgc tattaccatg gtgatgcggt tttggcagta catcaatggg 360
   cgtggatagc ggtttgactc acggggattt ccaagtctcc accccattga cgtcaatggg 420
   agtttgtttt ggcaccaaaa tcaacgggac tttccaaaat gtcgtaacaa ctccgcccca 480
   ttgacgcaaa tgggcggtag gcgtgtacgg tgggaggtct atataagcag agctcgttta 540
   gtgaaccgtc agatcgcctg gagacgccat ccacgctgtt ttgacctcca tagaagacac 600
   cgggaccgat ccagcctccg gactctagag gatccggtac tcgaggaact gaaaaaccag 660
   aaagttaact ggtaagttta gtctttttgt cttttatttc aggtcccgga tccggtggtg 720
   gtgcaaatca aagaactgct cctcagtgga tgttgccttt acttctaggc ctgtacggaa 780
   gtgttacttc tgctctaaaa gctgcggaat tgtacccgcg gccgctctgc catgggtcgg 840
   gggctgctcc ggggcctgtg gccgctgcac ctcgtcctgt ggacgcgcat cgccagcacg 900
   atcccgccgc acgttcagaa gtcggttaat aacgacatga tggtcacgga caacaatggc 960
   gccgtcaagt tcccacaact gtgcaagttt tgcgatgtgc gatcttccac ctgtgacaac 1020
   cagaagtcct gcatgagcaa ctgcagcatc acgtccatct gtgagaaggc acacgaagtc 1080
   tgcgtggccg tctggaggaa gaacgatgaa aacataaccc tggagactgt gtgtcacgac 1140
   cccaagctcg cctaccatgg attccttctg gaagattctg cctctccaaa gtgtatcatg 1200
   aaagaaaaga aggtgtttgg ggagactttc ttcatgtgtt cctgcagcac tgacgagtgc 1260
   aatgaccaca tcatcttctc tgaggaatac accaccagca gtccggatct ggtcgacaaa 1320
   actcacacat gcccaccgtg cccagcacct gaactcctgg ggggaccgtc agtcttcctc 1380
   ttccccccaa aacccaagga caccctcatg atctcccgga cccctgaggt cacatgcgtg 1440
   gtggtggacg tgagccacga agaccctgag gtcaagttca actggtacgt ggacggcgtg 1500
   gaggtgcata atgccaagac aaagccgcgg gaggagcagt acaacagcac gtaccgtgtg 1560
   gtcagcgtcc tcaccgtcct gcaccaggac tggctgaatg gcaaggagta caagtgcaag 1620
   gtctccaaca aagccctccc agcccccatc gagaaaacca tctccaaagc caaagggcag 1680
   ccccgagaac cacaggtgta caccctgccc ccatcccggg atgagctgac caagaaccag 1740
   gtcagcctga cctgcctggt caaaggcttc tatcccagcg acatcgccgt ggagtgggag 1800
   agcaatgggc agccggagaa caactacaag accacgcctc ccgtgttgga ctccgacggc 1860
   tccttcttcc tctacagcaa gctcaccgtg gacaagagca ggtggcagca ggggaacgtc 1920
   ttctcatgct ccgtgatgca tgaggctctg cacaaccact acacgcagaa gagcctctcc 1980
   ctgtctccgg gtaaatgagt gcggccgcgt cgaccgtgac ccctgcgccg cgcggactcc 2040
   tgccccgagg gtccggacgc gccccagctc gcgccccttc ccatatttat tcggacccca 2100
   agcatcgccc caataaagac cagcaagcaa ccggctgggg tgtccgtgcg tgttaggggg 2160
   cccgtgggac ctcccttgcc gtctctcctc gcgcacggcc cgggtccgcc ctgtagcgct 2220
   cgctgtctct cccctgcctg aagcgcccca ccaccgtctt tcaggccccg gacttggtgc 2280
   cgggtccagg cgtaaaggag caggtgactc tgcgcagcac tcgctttatt tcgccagagt 2340
   cgcggggcgt ccagaagggg cccctggact ccggcgcggg gccggctcag tcccgcccct 2400
   tgcctgcgcg gagcttctgg cgactcccag gccgctgctc ctcgtcggga cgcctcgggg 2460
   acacccaggc ctgcttcttc ctgggctcgg cgtccctgga gtcccgtgcc cacggccggt 2520
   ggcccccttc gcgtgactcc cagcgctggg gtagggcttc ccggggctcc ctggcggccc 2580
   gtggcctctc ctctttccgc ggcctctctt tcttaggtct ctcctccttc cgcggcttct 2640
   ccttccgcgg cttcccctct ctccgaggcc tctccttggg tccccggtct gcgagggtca 2700
   cacggtcctc ccggacggcc tctccggtgg cctcgccggg ctcctcttcg tcgttctctg 2760
   cagcctcccg actcccggga atcttgggtc tgacctctgc ctcggcccgg ggcgcaggcg 2820
   gcgctgatgg aggcgcctgg gcctcgaact taggctgcaa gacagagtgg ggtcctgggg 2880
   taagcgccca ccttcccccc ggcccgggct ccttctttcc ttggggatga aggtcccaat 2940
   gcccgcggtc agtggaagga agctcttacc aggggcgacg atggctccct cggggctgag 3000
   cttggcggga cccagggctc gggcacacgt gcttggagtg ctgcctccgc agggacggcg 3060
   tctgcagcga cagggtcggg acccgagtca gctgggccga ggcacggtcc acactgtcca 3120
   tccctccctc acccctagag cccccctccc tggacagttg gagtggggct cccccggtac 3180
   ggctgagact aaggatgccc ccgagccgtg ggaagggact ccgggactcc gctgccgcgg 3240
   cgccccttct ccgaacctcg ctctttcaat tggtcattct tccccccgac cacgggctgt 3300
   aggaggcccc tagcaaggga ggggtcgcag gagtgccccc ggggggcgct cacgagctga 3360
   gcggtcccca gagagggcgc aggggaaagg cggcagaacg ctacgaggca ggaggggttg 3420
   cacaaggttc atccggaagc cagaacctac tcgcggcgag gggaatgggc cccgcaaaag 3480
   gtccacaccg ggtgagaggg gcgcgcaagg cccgtcactt aagggacata tgacgtgagc 3540
   tcagatcttt gtgaaggaac cttacttctg tggtgtgaca taattggaca aactacctac 3600
   agagatttaa agctctaagg taaatataaa atttttaagt gtataatgtg ttaaactact 3660
   gattctaatt gtttgtgtat tttagattcc aacctatgga actgatgaat gggagcagtg 3720
   gtggaatgcc tttaatgagg aaaacctgtt ttgctcagaa gaaatgccat ctagtgatga 3780
   tgaggctact gctgactctc aacattctac tcctccaaaa aagaagagaa aggtagaaga 3840
   ccccaaggac tttccttcag aattgctaag ttttttgagt catgctgtgt ttagtaatag 3900
   aactcttgct tgctttgcta tttacaccac aaaggaaaaa gctgcactgc tatacaagaa 3960
   aattatggaa aaatattctg taacctttat aagtaggcat aacagttata atcataacat 4020
   actgtttttt cttactccac acaggcatag agtgtctgct attaataact atgctcaaaa 4080
   attgtgtacc tttagctttt taatttgtaa aggggttaat aaggaatatt tgatgtatag 4140
   tgccttgact agagatcata atcagccata ccacatttgt agaggtttta cttgctttaa 4200
   aaaacctccc acacctcccc ctgaacctga aacataaaat gaatgcaatt gttgttgtta 4260
   acttgtttat tgcagcttat aatggttaca aataaagcaa tagcatcaca aatttcacaa 4320
   ataaagcatt tttttcactg cattctagtt gtggtttgtc caaactcatc aatgtatctt 4380
   atcatgtctg gatcctctac gccggacgca tcgtggccgg catcaccggc gccacaggtg 4440
   cggttgctgg cgcctatatc gccgacatca ccgatgggga agatcgggct cgccacttcg 4500
   ggctcatgag cgcttgtttc ggcgtgggta tggtggcagg ccccgtggcc gggggactgt 4560
   tgggcgccat ctccttgcat gcaccattcc ttgcggcggc ggtgctcaac ggcctcaacc 4620
   tactactggg ctgcttccta atgcaggagt cgcataaggg agagcgtcga gactccatct 4680
   caaaaataaa ataaaataaa aattaaaaaa aaagggccct tgtgcaaagc tgacagcttg 4740
   tatgtttctg ctgttgacat ttgtgggctg tttaccaaca cttctggaac acagcagtgg 4800
   aagggacttc ccagatattt taaaattacc cttagaaagc ggtctgtgaa aaacccctac 4860
   ccaatttcct ttttgttaag tgacctaatt aacaggagga cacagagggt ggatgggcag 4920
   cctatgattg gaatgtcctc tcaagtagag gaggttaggg tttatgagga cacagaggag 4980
   cttcctgggg atccagacat gataagatac attgatgagt ttggacaaac cacaactaga 5040
   atgcagtgaa aaaaatgctt tatttgtgaa atttgtgatg ctattgcttt atttgtaacc 5100
   attataagct gcaataaaca agttaacaac aacaattgca ttcattttat gtttcaggtt 5160
   cagggggagg tgtgggaggt tttttaaagc aagtaaaacc tctacaaatg tggtatggct 5220
   gattatgatc tctagtcaag gcactataca tcaaatattc cttattaacc cctttacaaa 5280
   ttaaaaagct aaaggtacac aatttttgag catagttatt aatagcagac actctatgcc 5340
   tgtgtggagt aagaaaaaac agtatgttat gattataact gttatgccta cttataaagg 5400
   ttacagaata tttttccata attttcttgt atagcagtgc agctttttcc tttgtggtgt 5460
   aaatagcaaa gcaagcaaga gttctattac taaacacagc atgactcaaa aaacttagca 5520
   attctgaagg aaagtccttg gggtcttcta cctttctctt cttttttgga ggagtagaat 5580
   gttgagagtc agcagtagcc tcatcatcac tagatggcat ttcttctgag caaaacaggt 5640
   tttcctcatt aaaggcattc caccactgct cccattcatc agttccatag gttggaatct 5700
   aaaatacaca aacaattaga atcagtagtt taacacatta tacacttaaa aattttatat 5760
   ttaccttaga gctttaaatc tctgtaggta gtttgtccaa ttatgtcaca ccacagaagt 5820
   aaggttcctt cacaaagatc taaagccagc aaaagtccca tggtcttata aaaatgcata 5880
   gctttaggag gggagcagag aacttgaaag catcttcctg ttagtctttc ttctcgtaga 5940
   cttcaaactt atacttgatg cctttttcct cctggacctc agagaggacg cctgggtatt 6000
   ctgggagaag tttatatttc cccaaatcaa tttctgggaa aaacgtgtca ctttcaaatt 6060
   cctgcatgat ccttgtcaca aagagtctga ggtggcctgg ttgattcatg gcttcctggt 6120
   aaacagaact gcctccgact atccaaacca tgtctacttt acttgccaat tccggttgtt 6180
   caataagtct taaggcatca tccaaacttt tggcaagaaa atgagctcct cgtggtggtt 6240
   ctttgagttc tctactgaga actatattaa ttctgtcctt taaaggtcga ttcttctcag 6300
   gaatggagaa ccaggttttc ctacccataa tcaccagatt ctgtttacct tccactgaag 6360
   aggttgtggt cattctttgg aagtacttga actcgttcct gagcggaggc cagggtaggt 6420
   ctccgttctt gccaatcccc atattttggg acacggcgac gatgcagttc aatggtcgaa 6480
   ccatgatggc agcggggata aaatcctacc agccttcacg ctaggattgc cgtcaagttt 6540
   ggcgcgaaat cgcagccctg agctgtcccc ccccccaagc tttttgcaaa agcctaggcc 6600
   tccaaaaaag cctcctcact acttctggaa tagctcagag gccgaggcgg cctcggcctc 6660
   tgcataaata aaaaaaatta gtcagccatg gggcggagaa tgggcggaac tgggcggagt 6720
   taggggcggg atgggcggag ttaggggcgg gactatggtt gctgactaat tgagatgctg 6780
   cctcgcgcgt ttcggtgatg acggtgaaaa cctctgacac atgcagctcc cggagacggt 6840
   cacagcttgt ctgtaagcgg atgccgggag cagacaagcc cgtcagggcg cgtcagcggg 6900
   tgttggcggg tgtcggggcg cagccatgac ccagtcacgt agcgatagcg gagtgtatac 6960
   tggcttaact atgcggcatc agagcagatt gtactgagag tgcaccatat gcggtgtgaa 7020
   ataccgcaca gatgcgtaag gagaaaatac cgcatcaggc gctcttccgc ttcctcgctc 7080
   actgactcgc tgcgctcggt cgttcggctg cggcgagcgg tatcagctca ctcaaaggcg 7140
   gtaatacggt tatccacaga atcaggggat aacgcaggaa agaacatgtg agcaaaaggc 7200
   cagcaaaagg ccaggaaccg taaaaaggcc gcgttgctgg cgtttttcca taggctccgc 7260
   ccccctgacg agcatcacaa aaatcgacgc tcaagtcaga ggtggcgaaa cccgacagga 7320
   ctataaagat accaggcgtt tccccctgga agctccctcg tgcgctctcc tgttccgacc 7380
   ctgccgctta ccggatacct gtccgccttt ctcccttcgg gaagcgtggc gctttctcat 7440
   agctcacgct gtaggtatct cagttcggtg taggtcgttc gctccaagct gggctgtgtg 7500
   cacgaacccc ccgttcagcc cgaccgctgc gccttatccg gtaactatcg tcttgagtcc 7560
   aacccggtaa gacacgactt atcgccactg gcagcagcca ctggtaacag gattagcaga 7620
   gcgaggtatg taggcggtgc tacagagttc ttgaagtggt ggcctaacta cggctacact 7680
   agaaggacag tatttggtat ctgcgctctg ctgaagccag ttaccttcgg aaaaagagtt 7740
   ggtagctctt gatccggcaa acaaaccacc gctggtagcg gtggtttttt tgtttgcaag 7800
   cagcagatta cgcgcagaaa aaaaggatct caagaagatc ctttgatctt ttctacgggg 7860
   tctgacgctc agtggaacga aaactcacgt taagggattt tggtcatgag attatcaaaa 7920
   aggatcttca cctagatcct tttaaattaa aaatgaagtt ttaaatcaat ctaaagtata 7980
   tatgagtaaa cttggtctga cagttaccaa tgcttaatca gtgaggcacc tatctcagcg 8040
   atctgtctat ttcgttcatc catagttgcc tgactccccg tcgtgtagat aactacgata 8100
   cgggagggct taccatctgg ccccagtgct gcaatgatac cgcgagaccc acgctcaccg 8160
   gctccagatt tatcagcaat aaaccagcca gccggaaggg ccgagcgcag aagtggtcct 8220
   gcaactttat ccgcctccat ccagtctatt aattgttgcc gggaagctag agtaagtagt 8280
   tcgccagtta atagtttgcg caacgttgtt gccattgctg caggcatcgt ggtgtcacgc 8340
   tcgtcgtttg gtatggcttc attcagctcc ggttcccaac gatcaaggcg agttacatga 8400
   tcccccatgt tgtgcaaaaa agcggttagc tccttcggtc ctccgatcgt tgtcagaagt 8460
   aagttggccg cagtgttatc actcatggtt atggcagcac tgcataattc tcttactgtc 8520
   atgccatccg taagatgctt ttctgtgact ggtgagtact caaccaagtc attctgagaa 8580
   tagtgtatgc ggcgaccgag ttgctcttgc ccggcgtcaa cacgggataa taccgcgcca 8640
   catagcagaa ctttaaaagt gctcatcatt ggaaaacgtt cttcggggcg aaaactctca 8700
   aggatcttac cgctgttgag atccagttcg atgtaaccca ctcgtgcacc caactgatct 8760
   tcagcatctt ttactttcac cagcgtttct gggtgagcaa aaacaggaag gcaaaatgcc 8820
   gcaaaaaagg gaataagggc gacacggaaa tgttgaatac tcatactctt cctttttcaa 8880
   tattattgaa gcatttatca gggttattgt ctcatgagcg gatacatatt tgaatgtatt 8940
   tagaaaaata aacaaatagg ggttccgcgc acatttcccc gaaaagtgcc acctgacgtc 9000
   taagaaacca ttattatcat gacattaacc tataaaaata ggcgtatcac gaggcccttt 9060
   cgtcttcaag aattccg                                                9077
<210> 2
   <211> 1164
   <212> DNA
   <213> Rabbit
<220>
   <223> Description of Unknown Organism: Rabbit
<400> 2
   atgggtcggg ggctgctccg gggcctgtgg ccgctgcacc tcgtcctgtg gacgcgcatc 60
   gccagcacga tcccgccgca cgttcagaag tcggttaata acgacatgat ggtcacggac 120
   aacaatggcg ccgtcaagtt cccacaactg tgcaagtttt gcgatgtgcg atcttccacc 180
   tgtgacaacc agaagtcctg catgagcaac tgcagcatca cgtccatctg tgagaaggca 240
   cacgaagtct gcgtggccgt ctggaggaag aacgatgaaa acataaccct ggagactgtg 300
   tgtcacgacc ccaagctcgc ctaccatgga ttccttctgg aagattctgc ctctccaaag 360
   tgtatcatga aagaaaagaa ggtgtttggg gagactttct tcatgtgttc ctgcagcact 420
   gacgagtgca atgaccacat catcttctct gaggaataca ccaccagcag tccggatctg 480
   gtcgacaaaa ctcacacatg cccaccgtgc ccagcacctg aactcctggg gggaccgtca 540
   gtcttcctct tccccccaaa acccaaggac accctcatga tctcccggac ccctgaggtc 600
   acatgcgtgg tggtggacgt gagccacgaa gaccctgagg tcaagttcaa ctggtacgtg 660
   gacggcgtgg aggtgcataa tgccaagaca aagccgcggg aggagcagta caacagcacg 720
   taccgtgtgg tcagcgtcct caccgtcctg caccaggact ggctgaatgg caaggagtac 780
   aagtgcaagg tctccaacaa agccctccca gcccccatcg agaaaaccat ctccaaagcc 840
   aaagggcagc cccgagaacc acaggtgtac accctgcccc catcccggga tgagctgacc 900
   aagaaccagg tcagcctgac ctgcctggtc aaaggcttct atcccagcga catcgccgtg 960
   gagtgggaga gcaatgggca gccggagaac aactacaaga ccacgcctcc cgtgttggac 1020
   tccgacggct ccttcttcct ctacagcaag ctcaccgtgg acaagagcag gtggcagcag 1080
   gggaacgtct tctcatgctc cgtgatgcat gaggctctgc acaaccacta cacgcagaag 1140
   agcctctccc tgtctccggg taaa                                       1164
<210> 3
   <211> 9077
   <212> DNA
   <213> Rabbit
<400> 3
   gaattcgagc ttgcatgcct gcaggtcgtt acataactta cggtaaatgg cccgcctggc 60
   tgaccgccca acgacccccg cccattgacg tcaataatga cgtatgttcc catagtaacg 120
   ccaataggga ctttccattg acgtcaatgg gtggagtatt tacggtaaac tgcccacttg 180
   gcagtacatc aagtgtatca tatgccaagt acgcccccta ttgacgtcaa tgacggtaaa 240
   tggcccgcct ggcattatgc ccagtacatg accttatggg actttcctac ttggcagtac 300
   atctacgtat tagtcatcgc tattaccatg gtgatgcggt tttggcagta catcaatggg 360
   cgtggatagc ggtttgactc acggggattt ccaagtctcc accccattga cgtcaatggg 420
   agtttgtttt ggcaccaaaa tcaacgggac tttccaaaat gtcgtaacaa ctccgcccca 480
   ttgacgcaaa tgggcggtag gcgtgtacgg tgggaggtct atataagcag agctcgttta 540
   gtgaaccgtc agatcgcctg gagacgccat ccacgctgtt ttgacctcca tagaagacac 600
   cgggaccgat ccagcctccg gactctagag gatccggtac tcgaggaact gaaaaaccag 660
   aaagttaact ggtaagttta gtctttttgt cttttatttc aggtcccgga tccggtggtg 720
   gtgcaaatca aagaactgct cctcagtgga tgttgccttt acttctaggc ctgtacggaa 780
   gtgttacttc tgctctaaaa gctgcggaat tgtacccgcg gccgctctgc catgggtcgg 840
   gggctgctca ggggcctgtg gccgctgcac atcgtcctgt ggacgcgtat cgccagcacg 900
   atcccaccgc acgttcagaa gtcggttaat aacgacatga tagtcactga caacaacggt 960
   gcagtcaagt ttccacaact gtgtaaattt tgtgatgtga gattttccac ctgtgacaac 1020
   cagaaatcct gcatgagcaa ctgcagcatc acctccatct gtgagaagcc acaggaagtc 1080
   tgtgtggctg tatggagaaa gaatgacgag aacataacac tagagacagt ttgccatgac 1140
   cccaagctcc cctaccatga ctttattctg gaagatgctg cttctccaaa gtgcattatg 1200
   aaggaaaaaa aaaagcctgg tgagactttc ttcatgtgtt cctgtagctc tgatgagtgc 1260
   aatgacaaca tcatcttctc agaagaatat aacaccagca atcctgactt ggtcgacaaa 1320
   actcacacat gcccaccgtg cccagcacct gaactcctgg ggggaccgtc agtcttcctc 1380
   ttccccccaa aacccaagga caccctcatg atctcccgga cccctgaggt cacatgcgtg 1440
   gtggtggacg tgagccacga agaccctgag gtcaagttca actggtacgt ggacggcgtg 1500
   gaggtgcata atgccaagac aaagccgcgg gaggagcagt acaacagcac gtaccgtgtg 1560
   gtcagcgtcc tcaccgtcct gcaccaggac tggctgaatg gcaaggagta caagtgcaag 1620
   gtctccaaca aagccctccc agcccccatc gagaaaacca tctccaaagc caaagggcag 1680
   ccccgagaac cacaggtgta caccctgccc ccatcccggg atgagctgac caagaaccag 1740
   gtcagcctga cctgcctggt caaaggcttc tatcccagcg acatcgccgt ggagtgggag 1800
   agcaatgggc agccggagaa caactacaag accacgcctc ccgtgttgga ctccgacggc 1860
   tccttcttcc tctacagcaa gctcaccgtg gacaagagca ggtggcagca ggggaacgtc 1920
   ttctcatgct ccgtgatgca tgaggctctg cacaaccact acacgcagaa gagcctctcc 1980
   ctgtctccgg gtaaatgagt gcggccgcgt cgaccgtgac ccctgcgccg cgcggactcc 2040
   tgccccgagg gtccggacgc gccccagctc gcgccccttc ccatatttat tcggacccca 2100
   agcatcgccc caataaagac cagcaagcaa ccggctgggg tgtccgtgcg tgttaggggg 2160
   cccgtgggac ctcccttgcc gtctctcctc gcgcacggcc cgggtccgcc ctgtagcgct 2220
   cgctgtctct cccctgcctg aagcgcccca ccaccgtctt tcaggccccg gacttggtgc 2280
   cgggtccagg cgtaaaggag caggtgactc tgcgcagcac tcgctttatt tcgccagagt 2340
   cgcggggcgt ccagaagggg cccctggact ccggcgcggg gccggctcag tcccgcccct 2400
   tgcctgcgcg gagcttctgg cgactcccag gccgctgctc ctcgtcggga cgcctcgggg 2460
   acacccaggc ctgcttcttc ctgggctcgg cgtccctgga gtcccgtgcc cacggccggt 2520
   ggcccccttc gcgtgactcc cagcgctggg gtagggcttc ccggggctcc ctggcggccc 2580
   gtggcctctc ctctttccgc ggcctctctt tcttaggtct ctcctccttc cgcggcttct 2640
   ccttccgcgg cttcccctct ctccgaggcc tctccttggg tccccggtct gcgagggtca 2700
   cacggtcctc ccggacggcc tctccggtgg cctcgccggg ctcctcttcg tcgttctctg 2760
   cagcctcccg actcccggga atcttgggtc tgacctctgc ctcggcccgg ggcgcaggcg 2820
   gcgctgatgg aggcgcctgg gcctcgaact taggctgcaa gacagagtgg ggtcctgggg 2880
   taagcgccca ccttcccccc ggcccgggct ccttctttcc ttggggatga aggtcccaat 2940
   gcccgcggtc agtggaagga agctcttacc aggggcgacg atggctccct cggggctgag 3000
   cttggcggga cccagggctc gggcacacgt gcttggagtg ctgcctccgc agggacggcg 3060
   tctgcagcga cagggtcggg acccgagtca gctgggccga ggcacggtcc acactgtcca 3120
   tccctccctc acccctagag cccccctccc tggacagttg gagtggggct cccccggtac 3180
   ggctgagact aaggatgccc ccgagccgtg ggaagggact ccgggactcc gctgccgcgg 3240
   cgccccttct ccgaacctcg ctctttcaat tggtcattct tccccccgac cacgggctgt 3300
   aggaggcccc tagcaaggga ggggtcgcag gagtgccccc ggggggcgct cacgagctga 3360
   gcggtcccca gagagggcgc aggggaaagg cggcagaacg ctacgaggca ggaggggttg 3420
   cacaaggttc atccggaagc cagaacctac tcgcggcgag gggaatgggc cccgcaaaag 3480
   gtccacaccg ggtgagaggg gcgcgcaagg cccgtcactt aagggacata tgacgtgagc 3540
   tcagatcttt gtgaaggaac cttacttctg tggtgtgaca taattggaca aactacctac 3600
   agagatttaa agctctaagg taaatataaa atttttaagt gtataatgtg ttaaactact 3660
   gattctaatt gtttgtgtat tttagattcc aacctatgga actgatgaat gggagcagtg 3720
   gtggaatgcc tttaatgagg aaaacctgtt ttgctcagaa gaaatgccat ctagtgatga 3780
   tgaggctact gctgactctc aacattctac tcctccaaaa aagaagagaa aggtagaaga 3840
   ccccaaggac tttccttcag aattgctaag ttttttgagt catgctgtgt ttagtaatag 3900
   aactcttgct tgctttgcta tttacaccac aaaggaaaaa gctgcactgc tatacaagaa 3960
   aattatggaa aaatattctg taacctttat aagtaggcat aacagttata atcataacat 4020
   actgtttttt cttactccac acaggcatag agtgtctgct attaataact atgctcaaaa 4080
   attgtgtacc tttagctttt taatttgtaa aggggttaat aaggaatatt tgatgtatag 4140
   tgccttgact agagatcata atcagccata ccacatttgt agaggtttta cttgctttaa 4200
   aaaacctccc acacctcccc ctgaacctga aacataaaat gaatgcaatt gttgttgtta 4260
   acttgtttat tgcagcttat aatggttaca aataaagcaa tagcatcaca aatttcacaa 4320
   ataaagcatt tttttcactg cattctagtt gtggtttgtc caaactcatc aatgtatctt 4380
   atcatgtctg gatcctctac gccggacgca tcgtggccgg catcaccggc gccacaggtg 4440
   cggttgctgg cgcctatatc gccgacatca ccgatgggga agatcgggct cgccacttcg 4500
   ggctcatgag cgcttgtttc ggcgtgggta tggtggcagg ccccgtggcc gggggactgt 4560
   tgggcgccat ctccttgcat gcaccattcc ttgcggcggc ggtgctcaac ggcctcaacc 4620
   tactactggg ctgcttccta atgcaggagt cgcataaggg agagcgtcga gactccatct 4680
   caaaaataaa ataaaataaa aattaaaaaa aaagggccct tgtgcaaagc tgacagcttg 4740
   tatgtttctg ctgttgacat ttgtgggctg tttaccaaca cttctggaac acagcagtgg 4800
   aagggacttc ccagatattt taaaattacc cttagaaagc ggtctgtgaa aaacccctac 4860
   ccaatttcct ttttgttaag tgacctaatt aacaggagga cacagagggt ggatgggcag 4920
   cctatgattg gaatgtcctc tcaagtagag gaggttaggg tttatgagga cacagaggag 4980
   cttcctgggg atccagacat gataagatac attgatgagt ttggacaaac cacaactaga 5040
   atgcagtgaa aaaaatgctt tatttgtgaa atttgtgatg ctattgcttt atttgtaacc 5100
   attataagct gcaataaaca agttaacaac aacaattgca ttcattttat gtttcaggtt 5160
   cagggggagg tgtgggaggt tttttaaagc aagtaaaacc tctacaaatg tggtatggct 5220
   gattatgatc tctagtcaag gcactataca tcaaatattc cttattaacc cctttacaaa 5280
   ttaaaaagct aaaggtacac aatttttgag catagttatt aatagcagac actctatgcc 5340
   tgtgtggagt aagaaaaaac agtatgttat gattataact gttatgccta cttataaagg 5400
   ttacagaata tttttccata attttcttgt atagcagtgc agctttttcc tttgtggtgt 5460
   aaatagcaaa gcaagcaaga gttctattac taaacacagc atgactcaaa aaacttagca 5520
   attctgaagg aaagtccttg gggtcttcta cctttctctt cttttttgga ggagtagaat 5580
   gttgagagtc agcagtagcc tcatcatcac tagatggcat ttcttctgag caaaacaggt 5640
   tttcctcatt aaaggcattc caccactgct cccattcatc agttccatag gttggaatct 5700
   aaaatacaca aacaattaga atcagtagtt taacacatta tacacttaaa aattttatat 5760
   ttaccttaga gctttaaatc tctgtaggta gtttgtccaa ttatgtcaca ccacagaagt 5820
   aaggttcctt cacaaagatc taaagccagc aaaagtccca tggtcttata aaaatgcata 5880
   gctttaggag gggagcagag aacttgaaag catcttcctg ttagtctttc ttctcgtaga 5940
   cttcaaactt atacttgatg cctttttcct cctggacctc agagaggacg cctgggtatt 6000
   ctgggagaag tttatatttc cccaaatcaa tttctgggaa aaacgtgtca ctttcaaatt 6060
   cctgcatgat ccttgtcaca aagagtctga ggtggcctgg ttgattcatg gcttcctggt 6120
   aaacagaact gcctccgact atccaaacca tgtctacttt acttgccaat tccggttgtt 6180
   caataagtct taaggcatca tccaaacttt tggcaagaaa atgagctcct cgtggtggtt 6240
   ctttgagttc tctactgaga actatattaa ttctgtcctt taaaggtcga ttcttctcag 6300
   gaatggagaa ccaggttttc ctacccataa tcaccagatt ctgtttacct tccactgaag 6360
   aggttgtggt cattctttgg aagtacttga actcgttcct gagcggaggc cagggtaggt 6420
   ctccgttctt gccaatcccc atattttggg acacggcgac gatgcagttc aatggtcgaa 6480
   ccatgatggc agcggggata aaatcctacc agccttcacg ctaggattgc cgtcaagttt 6540
   ggcgcgaaat cgcagccctg agctgtcccc ccccccaagc tttttgcaaa agcctaggcc 6600
   tccaaaaaag cctcctcact acttctggaa tagctcagag gccgaggcgg cctcggcctc 6660
   tgcataaata aaaaaaatta gtcagccatg gggcggagaa tgggcggaac tgggcggagt 6720
   taggggcggg atgggcggag ttaggggcgg gactatggtt gctgactaat tgagatgctg 6780
   cctcgcgcgt ttcggtgatg acggtgaaaa cctctgacac atgcagctcc cggagacggt 6840
   cacagcttgt ctgtaagcgg atgccgggag cagacaagcc cgtcagggcg cgtcagcggg 6900
   tgttggcggg tgtcggggcg cagccatgac ccagtcacgt agcgatagcg gagtgtatac 6960
   tggcttaact atgcggcatc agagcagatt gtactgagag tgcaccatat gcggtgtgaa 7020
   ataccgcaca gatgcgtaag gagaaaatac cgcatcaggc gctcttccgc ttcctcgctc 7080
   actgactcgc tgcgctcggt cgttcggctg cggcgagcgg tatcagctca ctcaaaggcg 7140
   gtaatacggt tatccacaga atcaggggat aacgcaggaa agaacatgtg agcaaaaggc 7200
   cagcaaaagg ccaggaaccg taaaaaggcc gcgttgctgg cgtttttcca taggctccgc 7260
   ccccctgacg agcatcacaa aaatcgacgc tcaagtcaga ggtggcgaaa cccgacagga 7320
   ctataaagat accaggcgtt tccccctgga agctccctcg tgcgctctcc tgttccgacc 7380
   ctgccgctta ccggatacct gtccgccttt ctcccttcgg gaagcgtggc gctttctcat 7440
   agctcacgct gtaggtatct cagttcggtg taggtcgttc gctccaagct gggctgtgtg 7500
   cacgaacccc ccgttcagcc cgaccgctgc gccttatccg gtaactatcg tcttgagtcc 7560
   aacccggtaa gacacgactt atcgccactg gcagcagcca ctggtaacag gattagcaga 7620
   gcgaggtatg taggcggtgc tacagagttc ttgaagtggt ggcctaacta cggctacact 7680
   agaaggacag tatttggtat ctgcgctctg ctgaagccag ttaccttcgg aaaaagagtt 7740
   ggtagctctt gatccggcaa acaaaccacc gctggtagcg gtggtttttt tgtttgcaag 7800
   cagcagatta cgcgcagaaa aaaaggatct caagaagatc ctttgatctt ttctacgggg 7860
   tctgacgctc agtggaacga aaactcacgt taagggattt tggtcatgag attatcaaaa 7920
   aggatcttca cctagatcct tttaaattaa aaatgaagtt ttaaatcaat ctaaagtata 7980
   tatgagtaaa cttggtctga cagttaccaa tgcttaatca gtgaggcacc tatctcagcg 8040
   atctgtctat ttcgttcatc catagttgcc tgactccccg tcgtgtagat aactacgata 8100
   cgggagggct taccatctgg ccccagtgct gcaatgatac cgcgagaccc acgctcaccg 8160
   gctccagatt tatcagcaat aaaccagcca gccggaaggg ccgagcgcag aagtggtcct 8220
   gcaactttat ccgcctccat ccagtctatt aattgttgcc gggaagctag agtaagtagt 8280
   tcgccagtta atagtttgcg caacgttgtt gccattgctg caggcatcgt ggtgtcacgc 8340
   tcgtcgtttg gtatggcttc attcagctcc ggttcccaac gatcaaggcg agttacatga 8400
   tcccccatgt tgtgcaaaaa agcggttagc tccttcggtc ctccgatcgt tgtcagaagt 8460
   aagttggccg cagtgttatc actcatggtt atggcagcac tgcataattc tcttactgtc 8520
   atgccatccg taagatgctt ttctgtgact ggtgagtact caaccaagtc attctgagaa 8580
   tagtgtatgc ggcgaccgag ttgctcttgc ccggcgtcaa cacgggataa taccgcgcca 8640
   catagcagaa ctttaaaagt gctcatcatt ggaaaacgtt cttcggggcg aaaactctca 8700
   aggatcttac cgctgttgag atccagttcg atgtaaccca ctcgtgcacc caactgatct 8760
   tcagcatctt ttactttcac cagcgtttct gggtgagcaa aaacaggaag gcaaaatgcc 8820
   gcaaaaaagg gaataagggc gacacggaaa tgttgaatac tcatactctt cctttttcaa 8880
   tattattgaa gcatttatca gggttattgt ctcatgagcg gatacatatt tgaatgtatt 8940
   tagaaaaata aacaaatagg ggttccgcgc acatttcccc gaaaagtgcc acctgacgtc 9000
   taagaaacca ttattatcat gacattaacc tataaaaata ggcgtatcac gaggcccttt 9060
   cgtcttcaag aattccg                                                9077
<210> 4
   <211> 1165
   <212> DNA
   <213> Rabbit
<400> 4
   atgggtcggg ggctgctcag gggcctgtgg ccgctgcaca tcgtcctgtg gacgcgtatc 60
   gccagcacga tcccaccgca cgttcagaag tcggttaata acgacatgat agtcactgac 120
   aacaacggtg cagtcaagtt tccacaactg tgtaaatttt gtgatgtgag attttccacc 180
   tgtgacaacc agaaatcctg catgagcaac tgcagcatca cctccatctg tgagaagcca 240
   caggaagtct gtgtggctgt atggagaaag aatgacgaga acataacact agagacagtt 300
   tgccatgacc ccaagctccc ctaccatgac tttattctgg aagatgctgc ttctccaaag 360
   tgcattatga aggaaaaaaa aaagcctggt gagactttct tcatgtgttc ctgtagctct 420
   gatgagtgca atgacaacat catcttctca gaagaatata acaccagcaa tcctgacttg 480
   gtcgacaaaa ctcacacatg cccaccgtgc ccagcacctg aactcctggg gggaccgtca 540
   gtcttcctct tccccccaaa acccaaggac accctcatga tctcccggac ccctgaggtc 600
   acatgcgtgg tggtggacgt gagccacgaa gaccctgagg tcaagttcaa ctggtacgtg 660
   gacggcgtgg aggtgcataa tgccaagaca aagccgcggg aggagcagta caacagcacg 720
   taccgtgtgg tcagcgtcct caccgtcctg caccaggact ggctgaatgg caaggagtac 780
   aagtgcaagg tctccaacaa agccctccca gcccccatcg agaaaaccat ctccaaagcc 840
   aaagggcagc cccgagaacc acaggtgtac accctgcccc catcccggga tgagctgacc 900
   aagaaccagg tcagcctgac ctgcctggtc aaaggcttct atcccagcga catcgccgtg 960
   gagtgggaga gcaatgggca gccggagaac aactacaaga ccacgcctcc cgtgttggac 1020
   tccgacggct ccttcttcct ctacagcaag ctcaccgtgg acaagagcag gtggcagcag 1080
   gggaacgtct tctcatgctc cgtgatgcat gaggctctgc acaaccacta cacgcagaag 1140
   agcctctccc tgtctccggg taaat                                        1165
<210> 5
   <211> 39
   <212> DNA
   <213> Rabbit
<400> 5
   aaggaaaaaa gcggccgctc tgccatgggt cgggggctg 39
<210> 6
   <211> 34
   <212> DNA
   <213> Rabbit
<400> 6
   agttttgtcg accagatccg gactgctggg tggt 34
<210> 7
   <211> 34
   <212> DNA
   <213> Homo sapiens
<400> 7
   agttttgtcg accaagtcag gattgctggt gtta 34
<210> 8
   <211> 388
   <212> PRT
   <213> Rabbit
<400> 8
   Met Gly Arg Gly Leu Leu Arg Gly Leu Trp Pro Leu His Leu Val Leu
   1               5                   10                  15
   Trp Thr Arg Ile Ala Ser Thr Ile Pro Pro His Val Gln Lys Ser Val
               20                  25                  30
   Asn Asn Asp Met Met Val Thr Asp Asn Asn Gly Ala Val Lys Phe Pro
   35 40 45
   Gln Leu Cys Lys Phe Cys Asp Val Arg Ser Ser Thr Cys Asp Asn Gln
   50 55 60
   Lys Ser Cys Met Ser Asn Cys Ser Ile Thr Ser Ile Cys Glu Lys Ala
   65 70 75 80
   His Glu Val Cys Val Ala Val Trp Arg Lys Asn Asp Glu Asn Ile Thr
   85 90 95
   Leu Glu Thr Val Cys His Asp Pro Lys Leu Ala Tyr His Gly Phe Leu
   100 105 110
   Leu Glu Asp Ser Ala Ser Pro Lys Cys Ile Met Lys Glu Lys Lys Val
   115 120 125
   Phe Gly Glu Thr Phe Phe Met Cys Ser Cys Ser Thr Asp Glu Cys Asn
   130 135 140
   Asp His Ile Ile Phe Ser Glu Glu Tyr Thr Thr Ser Ser Pro Asp Leu
   145 150 155 160
   Val Asp Lys Thr His Thr Cys Pro Pro Cys Pro Ala Pro Glu Leu Leu
   165 170 175
   Gly Gly Pro Ser Val Phe Leu Phe Pro Pro Lys Pro Lys Asp Thr Leu
   180 185 190
   Met Ile Ser Arg Thr Pro Glu Val Thr Cys Val Val Val Asp Val Ser
   195 200 205
   His Glu Asp Pro Glu Val Lys Phe Asn Trp Tyr Val Asp Gly Val Glu
   210 215 220
   Val His Asn Ala Lys Thr Lys Pro Arg Glu Glu Gln Tyr Asn Ser Thr
   225 230 235 240
   Tyr Arg Val Val Ser Val Leu Thr Val Leu His Gln Asp Trp Leu Asn
   245 250 255
   Gly Lys Glu Tyr Lys Cys Lys Val Ser Asn Lys Ala Leu Pro Ala Pro
   260 265 270
   Ile Glu Lys Thr Ile Ser Lys Ala Lys Gly Gln Pro Arg Glu Pro Gln
   275 280 285
   Val Tyr Thr Leu Pro Pro Ser Arg Asp Glu Leu Thr Lys Asn Gln Val
   290 295 300
   Ser Leu Thr Cys Leu Val Lys Gly Phe Tyr Pro Ser Asp Ile Ala Val
   305 310 315 320
   Glu Trp Glu Ser Asn Gly Gln Pro Glu Asn Asn Tyr Lys Thr Thr Pro
   325 330 335
   Pro Val Leu Asp Ser Asp Gly Ser Phe Phe Leu Tyr Ser Lys Leu Thr
   340 345 350
   Val Asp Lys Ser Arg Trp Gln Gln Gly Asn Val Phe Ser Cys Ser Val
   355 360 365
   Met His Glu Ala Leu His Asn His Tyr Thr Gln Lys Ser Leu Ser Leu
   370 375 380
   Ser Pro Gly Lys
   385
<210> 9
   <211> 388
   <212> PRT
   <213> Homo sapiens
<400> 9
   Met Gly Arg Gly Leu Leu Arg Gly Leu Trp Pro Leu His Ile Val Leu
   1 5 10 15
   Trp Thr Arg Ile Ala Ser Thr Ile Pro Pro His Val Gln Lys Ser Val
   20 25 30
   Asn Asn Asp Met Ile Val Thr Asp Asn Asn Gly Ala Val Lys Phe Pro
   35 40 45
   Gln Leu Cys Lys Phe Cys Asp Val Arg Phe Ser Thr Cys Asp Asn Gln
   50 55 60
   Lys Ser Cys Met Ser Asn Cys Ser Ile Thr Ser Ile Cys Glu Lys Pro
   65 70 75 80
   Gln Glu Val Cys Val Ala Val Trp Arg Lys Asn Asp Glu Asn Ile Thr
   85 90 95
   Leu Glu Thr Val Cys His Asp Pro Lys Leu Pro Tyr His Asp Phe Ile
   100 105 110
   Leu Glu Asp Ala Ala Ser Pro Lys Cys Ile Met Lys Glu Lys Lys Lys
   115 120 125
   Pro Gly Glu Thr Phe Phe Met Cys Ser Cys Ser Ser Asp Glu Cys Asn
   130 135 140
   Asp Asn Ile Ile Phe Ser Glu Glu Tyr Asn Thr Ser Asn Pro Asp Leu
   145 150 155 160
   Val Asp Lys Thr His Thr Cys Pro Pro Cys Pro Ala Pro Glu Leu Leu
   165 170 175
   Gly Gly Pro Ser Val Phe Leu Phe Pro Pro Lys Pro Lys Asp Thr Leu
   180 185 190
   Met Ile Ser Arg Thr Pro Glu Val Thr Cys Val Val Val Asp Val Ser
   195 200 205
   His Glu Asp Pro Glu Val Lys Phe Asn Trp Tyr Val Asp Gly Val Glu
   210 215 220
   Val His Asn Ala Lys Thr Lys Pro Arg Glu Glu Gln Tyr Asn Ser Thr
   225 230 235 240
   Tyr Arg Val Val Ser Val Leu Thr Val Leu His Gln Asp Trp Leu Asn
   245 250 255
   Gly Lys Glu Tyr Lys Cys Lys Val Ser Asn Lys Ala Leu Pro Ala Pro
   260 265 270
   Ile Glu Lys Thr Ile Ser Lys Ala Lys Gly Gln Pro Arg Glu Pro Gln
   275 280 285
   Val Tyr Thr Leu Pro Pro Ser Arg Asp Glu Leu Thr Lys Asn Gln Val
   290 295 300
   Ser Leu Thr Cys Leu Val Lys Gly Phe Tyr Pro Ser Asp Ile Ala Val
   305 310 315 320
   Glu Trp Glu Ser Asn Gly Gln Pro Glu Asn Asn Tyr Lys Thr Thr Pro
   325 330 335
   Pro Val Leu Asp Ser Asp Gly Ser Phe Phe Leu Tyr Ser Lys Leu Thr
   340 345 350
   Val Asp Lys Ser Arg Trp Gln Gln Gly Asn Val Phe Ser Cys Ser Val
   355 360 365
   Met His Glu Ala Leu His Asn His Tyr Thr Gln Lys Ser Leu Ser Leu
   370 375 380
   Ser Pro Gly Lys
   385
<210> 10
   <211> 66
   <212> DNA
   <213> Rabbit
<400> 10
   tctgaggaat acaccaccag cagtccggat ctggtcgaca aaactcacac atgcccaccg 60
   tgccca 66
<210> 11
   <211> 22
   <212> PRT
   <213> Rabbit
<400> 11
   Ser Glu Glu Tyr Thr Thr Ser Ser Pro Asp Leu Val Asp Lys Thr His
   1 5 10 15
   Thr Cys Pro Pro Cys Pro
   20
<210> 12
   <211> 68
   <212> DNA
   <213> Homo sapiens
<400> 12
   tctcagaaga atataacacc agcaatcctg acttggtcga caaaactcac acatgcccac 60
   cgtgccca 68
<210> 13
   <211> 22
   <212> PRT
   <213> Homo sapiens
<400> 13
   Ser Glu Glu Tyr Asn Thr Ser Asn Pro Asp Leu Val Asp Lys Thr His
   1 5 10 15
   Thr Cys Pro Pro Cys Pro
   20

## Description

### BACKGROUND OF THE INVENTION

The present invention relates to soluble TGF-beta receptor fusion proteins and to their uses in methods of treating conditions characterized by an overproduction of TGF-beta, including fibroproliferation.

### BACKGROUND

Transforming growth factor-beta (TGF-beta) represents a family of polypeptides, of which three are present in mammals, TGF-beta 1. TGF-beta 2 and TGF-beta 3. These factors have global effects on cell growth and differentiation (Roberts and Sporn (1990) Handok. Exp. Pharm. 95:419-58). There is a growing body of evidence that TGF-beta also modulates the immune process (Wahl et al. (1989) Immunol. Today 10:258-61). In addition to stimulating the congregation of immune cells at the site of injury, TGF-beta also provides strong positive feedback for its own continued synthesis (Kim et al. (1990) Mol.Cell. Biol. 10:1492-1497).

TGF-beta may be a prominent factor in the etiology of fibroproliferative disorders. TGF-beta is known to stimulate cells to produce more proteins, including collagen, biglycan, decorin and fibronectin: and to inhibit enzymes which degrade these proteins. Thus, TGF-beta can cause fibrous tissue to accumulate. For example, in diabetic nephropathy and human mesangial proliferative glomerulonephritis, both fibroproliferative disorders, a prominent and important pathological feature is the accumulation of mesangial matrix (Mauer et al. (1984) J. Clin. Invest, 74: 1143-55). Likewise, postradiation fibrosis is characterized by excess TGF-beta, proliferation of fibroblasts and overproduction of connective tissue (Canney and Dean (1990) Brit. J. Radiol. 63:620-23). Workers in the field have attempted to suppress the effects of TGF-beta by administering anti-TGF-beta antibody. See Border et al. (1990) Nature 346:371-74; Border et al. ((1992) Kidney Int. 41:566-570).

The biological effects of TGF-beta are mediated by several specific cell surface proteins, including the so-called type L type II, and type III TGF-beta receptors. Most of these were initially identified by chemically crosslinking radio iodinated TGF-beta to cell surface proteins using the bifunctional reagent DSS. Almost all TGF-beta responsive cell lines express both the type I and type II receptors, although the levels of their expression may vary among different cell types. Neither type I nor type II receptor in the absence of the other can mediate a TGF-beta response, although the type II receptor alone is capable of binding TGF-beta.

In developing improved TGF-beta inhibitors to treat fibroproliferative disorders, it is important to consider that such inhibitors should have a much lower molecular weight and higher affinity than anti-TGF-beta antibodies. This combination of features would permit much lower doses and increase ease of administration. Moreover, a native protein would not cause cross-species reactions.

### SUMMARY OF THE INVENTION

Accordingly, the present invention is directed to novel compositions and uses of compositions in methods for the therapeutic treatment of patients having fibroproliferative disorders as described hereinbelow.

The invention is also directed to recombinant DNA vectors which, allow expression of TGF-beta receptor fusion proteins in bacterial and mammalian host cells and methods of use thereof.

One aspect of the invention is an isolated TGF-beta receptor fusion protein that competitively inhibits binding of TGF-beta to TGF-beta receptor, wherein this protein is a TGF-beta Type II receptor comprising (a) a polypeptide encoded by the polypeptide shown in SEQ ID NO: 2; (b) a polypeptide comprising the amino acid sequence of SEQ ID NO: 8; or (c) a polypeptide comprising an amino acid sequence which is at least 98% identical to the amino acid sequence of SEQ ID NO: 8 linked to a second protein that is not a TGF-beta Type II receptor in which the second protein is a constant region of an immunoglobulin. Other preferred proteins contain the novel amino acid sequence of SEQ ID NO: 11 which encompasses the region of the fusion on either side of the TGF-beta receptor/immunoglobulin junction.

Pharmaceutical compositions of the invention are exemplified by a TGF-beta receptor fusion protein comprising (a) a polypeptide encoded by the polypeptide shown in SEQ ID NO: 2; (b) a polypeptide comprising the amino acid sequence of SEQ ID NO: 8; or (c) a polypeptide comprising an amino acid sequence which is at least 98% identical to the amino acid sequence of SEQ ID NO: 8 in a pharmaceutically acceptable carrier, the fusion protein in an amount sufficient to competitively inhibit binding of TGF-beta to a TGF-beta ligand.

In another aspect of the invention relates to the use of a TGF-beta RII fusion protein comprising
(a) a polypeptide encoded by the polynucleotide shown in SEQ ID NO: 2 or 4;
(b) a polypeptide comprising the amino acid sequence of SEQ ID NO: 8 or 9; or
(c) a polypeptide comprising an amino acid sequence which is at least 98% , identical to the amino acid sequence of SEQ ID NO: 8 or 9,
and a constant region of an immunoglobulin, wherein said TGF-beta RII fusion protein binds TGF-beta for the preparation of a pharmaceutical composition for the treatment of a subject suffering from a fibroproliferative disorder.

Isolated polynucleotides are also embodied herein, encoding, on expression, for an TGF-beta Type II receptor linked to a second protein that is not a TGF-beta Type II receptor as described herein. The preferred polynucleotides are selected from the group consisting of SEQ ID NOS: 1, 2, 3, 4, 10 and 12. Polynucleotides that hybridize to these sequences under standard hybridization conditions and that encodes a protein having the TGF-beta inhibitory activity of a TGF-beta Type II receptor fusion protein are also described herein. Polynucleotides that code on expression for a protein encoded by the preferred polynucleotide sequences are also embodied herein.

Methods of the invention include a method for producing the TGF-beta receptor fusion protein as described herein comprising culturing a host cell containing a vector of the invention, allowing said cell to express the fusion protein, isolating and purifying the fusion protein.

One embodiment is a use of a composition in a method for lowering the levels of TGF-beta in an individual which comprises administering to the individual an effective amount of a TGF-beta antagonist that is a TGF-beta receptor fusion protein comprising the sequence of amino acids of SEQ ID NOS: 8, 9, 11 and 13.

Another embodiment is use of a composition in a method for lowering the levels of TGF-beta in an individual having arthritis, which comprises administering to the individual an effective amount of a TGF-beta antagonist that a TGF-beta receptor fusion protein comprising the sequence of amino acids of SEQ ID NOS: 8, 9, 11 and 13. In one embodiment, the invention provides a use of a composition in a method for treating an individual for medical condition associated with a fibioproliferative disorder. The method provides for the parenteral, oral or local administration of a sufficient arrrount of soluble TGF-beta - receptor fusion protein to the individual to reduce or ameliorate the disorder and/or block activity of TGF-beta activity in the individual.

In yet another embodiment, the use of the composition in the method of the present invention provides for TGF-beca receptor fusion protein administration by intravenous, intraocular, intraarticular, transdermal and enteral methods. In another embodiment of the present invention, the composition comprising the TGF-beta receptor fusion protein is administered to patients with collagen vascular disorders, such as systemic sclerosis, polymyositis, scleroderma, dermatomyositis, or systemic lupus erythematosus.

In yet another embodiment of the present invention, the composition comprising the TGF-beta receptor fusion protein is administered to patients with with intraocular and pulmonary fibrosis. In a further embodiment, the TGF-beta receptor fusion protein is administered to patients with diabetic
nephropathy, glomerulonephritis, proliferative vitreoretinopathy, and rheumatoid arthritis.

In still another embodiment, the use of the composition in the method of the present invention provides for treating a wound in an individual to avoid overproduction of connective tissue formation which is associated with upregulation of TGF-beta. The use of the composition of the method provides for administration of a sufficient amount of TGF-beta receptor fusion protein to the individual to reduce the amount or activity of TGF-beta in the individual. In further embodiments, the types of wounds include surgical incisions, trauma-induced lacerations and wounds involving the peritoneum for which the overproduction of connective tissue formation is abdominal adhesions. In a further embodiment, the overproduction of connective tissue formations which are avoided include scars, including those where the scar involves restenosis of blood vessels, and hypertrophic scars, and keloids.

In another embodiment, the invention provides a use of a composition in a method of preventing postradiation fibrosis in an individual undergoing or about to undergo radiation therapy. TGF-beta receptor fusion protein is administered in an amount sufficient to prevent overproduction of fibrous tissue formation.

It is to be understood that both the foregoing general description and the following detailed description are exemplary and explanatory and are intended, to provide further explanation of the invention as claimed.

### LIST OF THE SEQUENCES

**SEQ ID NO: 1 Nucleotide Sequence of the Rabbit TGFβRII:Fc Expression Vector**
**Sequence ID #2: Nucleotide sequence of the rabbit TGFβRII:Fc**
**SEQUENCE ID #3: Nucleotide Sequence of the human TGFβRII:Fc expression vector**
**SEQUENCE ID# 4: Nucleotide sequence of human TGFβRII:Fc**
**SEQUENCE ID #5: Forward Primer -Rabbit and Human**
   5' - AAGGAAAAAAGCGGCCGCTCTGCCATGGGTCGGGGGCTG - 3'
**SEQUENCE ID #6: Reverse Primer - Rabbit**
   5' - AGTTTTGTCGACCAGATCCGGACTGCTGGGTGGT - 3'
**SEQUENCE ID #7: Reverse Primer - Human**
   5' - AGTTTTGTCGACCAAGTCAGGATTGCTGGTGTTA - 3'
**Sequence ID #8: Amino acid sequence of the rabbit TGFβRII:Fc**
**SEQUENCE ID# 9: Amino acid sequence of human TGFβRII:Fc**
**Sequence ID No. 10: Rabbit RII:Fc fusion "Junction" nucleotide sequence**
   TCTGAGGAATACACCACCAGCAGTCCGGATCTGGTCGACAAAACTCACACATGCCCACCGTGCCCA
**SEQ ID NO: 11 Rabbit RII:Fc fusion "Junction" amino acid sequence**
   S E E Y T T S S P D L V D K T H T C P P C P
**Sequence ID No. 12: Human RII:Fc fusion "Junction" nucleotide sequence**
   TCTCAGAAGAATATAACACCAGCAATCCTGACTTGGTCGACAAAACTCACACATGCCCACCGTGC CCA
**Sequence ID No. 13: Human RII:Fc fusion "Junction" amino acid sequence**
   S E E Y N T S N P D L V D K T H T C P P C P

### DETAILED DESCRIPTION

The following terms are used herein:

### Definitions

"Recombinant," as used herein, means that a protein is derived from recombinant (e.g., microbial or mammalian) expression systems. "Microbial" refers to recombinant proteins made in bacterial or fungal (e.g., yeast) expression systems. As a product. "recombinant microbial" defines a protein produced in a microbial expression system which is essentially free of native endogenous substances. Protein expressed in most bacterial cultures, e.g., E. coli, will be free of glycan. Protein expressed in yeast may have a glycosylation pattern different from that expressed in mammalian cells.

"Biologically active," as used throughout the specification as a characteristic of TGF-beta receptors, means that a particular molecule shares sufficient amino acid sequence homology with the embodiments of the present invention disclosed herein to be capable of binding detectable quantities of TGF-beta and/or transmitting a TGF-beta stimulus to a cell, for example, as a component of a hybrid receptor construct, or cross-reacting with anti-TGF-beta receptor antibodies raised against TGF-beta receptor from natural (i.e., nonrecombinant) sources. Preferably, biologically active TGF-beta receptor fusions within the scope of the present invention are capable of binding greater than 0.1 nmoles TGF-beta per nmole receptor, and most preferably, greater than 0.5 nmole TGF-beta per nmole receptor in standard binding assays (see below).

Similarly, a "TGF-beta binding molecule", as used herein, is any molecule exhibiting the same, or substantially the same, biological activity as a TGF-beta receptor fusion protein.

"Individual" means a living organism, including humans, other mammals and any other animals which produce TGF-beta.

"TGF-beta overproduction" as used herein is an amount of TGF-beta present in serum or tissue which is significantly above the normal level. More preferably, TGF-beta overproduction is a level between about 2 and about 20 times normal. Even more preferably, TGF-beta overproduction is a level between about 2 and about 15 times normal. For example, Deguchi measured 24-hour TGF-beta production of bronchoalveolar cells and reported normal levels of 410 +/- 225 pg/10⁷ cells
against excess TGF-beta production of 1288 +/- 453 pg/10⁷ cells in systemic lupus erythematosus and 1417 +/- 471 pg/10⁷ cells in scleroderma ((1992) Ann. Rheum. Dis. 51:362-65). TGF-beta overproduction can be determined, in combination with normal levels, by measurement of the TGF-beta protein, of TGF-beta MRNA, or of products whose synthesis is stimulated by TGF-beta , such as collagen.

"Connective tissue" is fibrous tissue characterized by the presence of fibroblasts and fibrous proteins such as collagen and elastin.

A"fibroproliferative disorder" is characterized by proliferation of fibroblasts plus the corresponding overexpression of extracellular matrix such as fibronectin, laminin and collagen.

A "therapeutic composition" as used herein is defined as comprising the proteins of the invention and other physiologically compatible ingredients. The therapeutic composition may contain excipients such as water, minerals and carriers such as protein.

"A sufficient amount" of the proteins of the invention as used herein refers to the amount of TGF-beta receptor fusion protein that neutralizes the biologic activity of excess TGF-beta . It may be determined by (1) suitable clinical variables of improvement, (2) pathologic evaluation of the effects on fibrosis and/or immunosuppression or prevention of fibrosis, or (3) a direct inhibition of TGF-beta.

"amino acid"- a monomeric unit of a peptide, polypeptide, or protein. There are twenty amino acids found in naturally occurring peptides, polypeptides and proteins, all of which are L-isomers. The term also includes analogs of the amino acids and D-isomers of the protein amino acids and their analogs.

"protein"- any polymer consisting essentially of any of the 20 amino acids. Although "polypeptide" is often used in reference to relatively large polypeptides, and "peptide" is often used in reference to small polypeptides, usage of these terms in the art overlaps and is varied. The term "protein" as used herein refers to peptides, proteins and polypeptides, unless otherwise noted.

"functional equivalent" of an amino acid residue is an amino acid having similar physico-chemical properties as the amino acid residue that was replaced by the functional equivalent.

"fusion"- refers to a co-linear, covalent linkage of two or more proteins or fragments thereof via their individual peptide backbones, most preferably through genetic expression of a polynucleotide molecule encoding those proteins.

"mutant" - any change in the genetic material of an organism, in particular any change (i.e., deletion, substitution, addition, or alteration) in a wild-type polynucleotide sequence or any change in a wild-type protein.

"wild-type" - the naturally-occurring polynucleotide sequence of an exon of a protein, or a portion thereof, or protein sequence, or portion thereof, respectively, as it normally exists *in vivo.*

"standard hybridization conditions"- salt and temperature conditions substantially equivalent to 0.5 X SSC to about 5 X SSC and 65 ° C for both hybridization and wash. The term "standard hybridization conditions" as used herein is therefore an operational definition and encompasses a range of hybridization conditions. Higher stringency conditions may, for example, include hybridizing with plaque screen buffer (0.2% polyvinylpyrrolidone, 0.2% Ficoll 400; 0.2% bovine serum albumin, 50 mM Tris-HCl (pH 7.5); 1 M NaCl; 0.1% sodium pyrophosphate; 1 % SDS); 10% dextran sulphate, and 100 µg/ml denatured, sonicated salmon sperm DNA at 65 ° C for 12-20 hours, and washing with 75 mM NaCl/7.5 mM sodium citrate (0.5 x SSC)/1% SDS at 65° C. Lower stringency conditions may, for example, include hybridizing with plaque screen buffer, 10% dextran sulphate and 110 µg/ml denatured, sonicated salmon sperm DNA at 55 ° C for 12-20 hours, and washing with 300 mM NaCl/30mM sodium citrate (2.0 X SSC)/1% SDS at 55 ° C. See also Current Protocols in Molecular Biology, John Wiley & Sons, Inc. New York, Sections 6.3.1-6.3.6, (1989).

"expression control sequence"- a sequence of polynucleotides that controls and regulates expression of genes when operatively linked to those genes.

"operatively linked"- a polynucleotide sequence (DNA, RNA) is operatively linked to an expression control sequence when the expression control sequence controls and regulates the transcription and translation of that polynucleotide sequence. The term "operatively linked" includes having an appropriate start signal (e.g., ATG) in front of the polynucleotide sequence to be expressed and maintaining the correct reading frame to permit expression of the polynucleotide sequence under the control of the expression control sequence and production of the desired polypeptide encoded by the polynucleotide sequence.

"expression vector"- a polynucleotide, such as a DNA plasmid or phage (among other common examples) which allows expression of at least one gene when the expression vector is introduced into a host cell. The vector may, or may not, be able to replicate in a cell.

"Isolated" (used interchangeably with "substantially pure")- when applied to nucleic acid i.e., polynucleotide sequences, that encode polypeptides, means an RNA or DNA polynucleotide, portion of genomic polynucleotide, cDNA or synthetic polynucleotide which, by virtue of its origin or manipulation: (i) is not associated with all of a polynucleotide with which it is associated in nature (e.g., is present in a host cell as an expression vector, or a portion thereof); or (ii) is linked to a nucleic acid or other chemical moiety other than that to which it is linked in nature; or (iii) does not occur in nature. By "isolated" it is further meant a polynucleotide sequence that is: (i) amplified *in vitro* by, for example, polymerase chain reaction (PCR); (ii) chemically synthesized; (iii) recombinantly produced by cloning; or (iv) purified, as by cleavage and gel separation.

Thus, "substantially pure nucleic acid" is a nucleic acid which is not immediately contiguous with one or both of the coding sequences with which it is normally contiguous in the naturally occurring genome of the organism from which the nucleic acid is derived. Substantially pure DNA also includes a recombinant DNA which is part of a hybrid gene encoding additional sequences.

"Isolated" (used interchangeably with "substantially pure")- when applied to polypeptides means a polypeptide or a portion thereof which, by virtue of its origin or manipulation: (i) is present in a host cell as the expression product of a portion of an expression vector; or (ii) is linked to a protein or other chemical moiety other than that to which it is linked in nature; or (iii) does not occur in nature. By "isolated" it is further meant a protein that is : (i) chemically synthesized; or (ii) expressed in a host cell and purified away from associated proteins. The term generally means a polypeptide that has been separated from other proteins and nucleic acids with which it naturally occurs. Preferably, the polypeptide is also separated from substances such as antibodies or gel matrices (polyacrylamide) which are used to purify it.

"heterologous promoter"- as used herein is a promoter which is not naturally associated with a gene or a purified nucleic acid.

"Homologous"- as used herein is synonymous with the term "identity" and refers to the sequence similarity between two polypeptides, molecules or between two nucleic acids. When a position in both of the two compared sequences is occupied by the same base or amino acid monomer subunit (for instance, if a position in each of the two DNA molecules is occupied by adenine, or a position in each of two polypeptides is occupied by a lysine), then the respective molecules are homologous at that position. The percentage homology between two sequences is a function of the number of matching or homologous positions shared by the two sequences divided by the number of positions compared x 100. For instance, if 6 of 10 of the positions in two sequences are matched or are homologous, then the two sequences are 60% homologous. By way of example, the DNA sequences CTGACT and CAGGTT share 50% homology (3 of the 6 total positions are matched). Generally, a comparison is made when two sequences are aligned to give maximum homology. Examples of references to DEFINE??

The terms "peptide(s)", "protein(s)" and "polypeptide(s)" are used interchangeably herein. The terms "polynucleotide sequence" and "nucleotide sequence" are also used interchangeably herein.

Practice of the present invention will employ, unless indicated otherwise, conventional techniques of cell biology, cell culture, molecular biology, microbiology, recombinant DNA, protein chemistry, and immunology, which are within the skill of the art. Such techniques are described in the literature. See, for example, Molecular Cloning: A Laboratory Manual, 2nd edition. (Sambrook, Fritsch and Maniatis, eds.). Cold Spring Harbor Laboratory Press. 1989; DNA Cloning, Volumes I and II (D.N. Glover, ed), 1985: Oligonucleotide Synthesis. (M.J. Gait, ed.), 1984; U.S. Patent No. 4,683,195 (Mullis et al.,); Nucleic Acid Hybridization (B.D. Hames and S.J. Higgins, eds.), 1984; Transcription and Translation (B.D. Hames and S.J. Higgins, eds.), 1984; Culture of Animal Cells (R.I. Freshney, ed). Alan R. Liss. Inc., 1987: Immobilized Cells and Enzymes, IRL Press, 1986; A Practical Guide to Molecular Cloning (B. Perbal), 1984; Methods in Enzymology, Volumes 154 and 155 (Wu et al., eds), Academic Press. New York; Gene Transfer Vectors for Mammalian Cells (J.H. Miller and M.P. Calos, eds.), 1987, Cold Spring Harbor Laboratory; Immunochemical Methods in Cell and Molecular Biology (Mayer and Walker, eds.). Academic Press, London, 1987; Handbook of Experiment Immunology, Volumes I-IV (D.M. Weir and C.C. Blackwell, eds.), 1986: Manipulating the Mouse Embryo, Cold Spring Harbor Laboratory Press, 1986.

### II. General Properties of Isolated Proteins and Polynucleotides

### A. Soluble TGF-beta receptor fusions and their Analogs as TGF-beta antagonists

The present invention utilizes isolated and purified TGF-beta antagonist polypeptides. The isolated and purified TGF-beta antagonist polypeptides used in this invention are substantially free of other contaminating materials of natural or endogenous origin and contain less than about 1% by mass of protein contaminants residual of production processes. The TGF-beta antagonist polypeptides used in this invention are optionally without associated native-pattern glycosylation.

As used herein, the term "TGF-beta receptor" refers to proteins having amino acid sequences which are substantially similar to native mammalian TGF-beta receptor or TGF-beta binding molecule sequences, and which are capable of binding TGF-beta and inhibiting TGF-beta from binding to cell membranes containing TGF-beta receptor. The preferred TGF-beta receptor is mature full-length TGF-beta receptor Type II. TGF-beta receptor Type II is a membrane-bound protein with an intracellular
domain, transmembrane domain and extracellular portion which binds to TGF-beta Human TGF-beta receptor Type II has been determined to have the amino acid sequence shown in Lin et al., Cell: 68, 775-785 (1992), and corrected as shown in U.S. Patent 5,693,607.

The preferred TGF-beta receptors of the present invention are soluble forms of TGF-beta receptor Type II, as well as soluble TGF-beta Type II binding molecules. Soluble TGF-beta receptors include, for example, analogs or subunits of native proteins having at least 20 amino acids and which exhibit at least some biological activity in common with TGF-beta receptor Type II or TGF-beta binding molecules.

The terms "TGF-beta Type II receptor fusion protein" or "TGF-beta RII:Fc" are used interchangeably and represent the most preferred embodiments of the compositions of the present invention. This fusion protein contains at least part of the TGF-beta receptor (Type II) linked to at least part of a second protein that is not TGF-beta receptor Type II. Specifically, the second protein may be the contant region of an immunoglobulin (preferably IgG, most preferably IgG1) or may be a portion thereof such as the hinge, C_{H}2 or C_{H}3. The preferred fusions of the invention are homodimers, connected to each other by disulfide linkages but heterodimeric forms are also means to be within the scope of the invention. See Section IV.

Thus, the most preferred compositions of the present invention are TGF-beta antagonists that are soluble fusion proteins such as recombinant chimeric antibody molecules having TGF-beta receptor sequences substituted for the variable domains of either or both of the immunoglobulin molecule heavy and light chains and having unmodified constant region domains. For example, chimeric TGF-betaRII/IgG 1 is produced from a chimeric genes: a TGF-beta RII/human gamma 1 heavy chain chimera. Following transcription and translation of the chimeric gene, the gene product assembles into a homodimer having TGF-beta receptor displayed bivalently. Such polyvalent forms of TGF-beta receptor may have enhanced binding affinity for TGF-beta ligand.

The soluble TGF-beta receptor constructs of the invention are devoid of a transmembrane region (and are secreted from the cell) but retain the ability to bind TGF-beta. Various bioequivalent protein and amino acid analogs have an amino acid sequence corresponding to all or part of the extracellular region of a native TGF-beta receptor (e.g. SEQ ID NO: 8 or 9 or amino acid sequences substantially similar or are at least 98% homologous to the sequences of SEQ ID NO: 8 or 9 and which are biologically active in that they bind to TGF-beta ligand. Equivalent TGF-beta receptors include polypeptides which vary from these sequences by one or more substitutions, deletions, or additions, and which retain the ability to bind TGF-beta or inhibit TGF-beta signal transduction activity via cell surface bound TGF-beta receptor proteins Inhibition of TGF-beta signal transduction activity can be determined by transfecting cells with recombinant TGF-beta receptor DNAs to obtain recombinant receptor expression. The cells are then contacted with TGF-beta and the resulting metabolic effects examined. If an effect results which is attributable to the action of the ligand, then the recombinant receptor has signal transduction activity. Exemplary procedures for determining whether a polypeptide has signal transduction activity are disclosed by Idzerda et al., J. Exp. Med. 171:861 (1990); Curtis et al., Proc. Natl. Acad. Sci.U.S.A. 86:3045 (1989); Prywes et al., EMBO J. 5:2179 (1986) and Chou et al., J. Biol. Chem. 262:1842 (1987). Alternatively, primary cells or cell lines which express an endogenous TGF-beta receptor and have a detectable biological response to TGF-beta could also be utilized.

A "fragment" of the proteins of this invention is a portion or all of a protein which is capable of binding TGF-beta. Preferably, this fragment has a high affinity for TGF-beta. Preferred fragments of fusion proteins are protein fragments comprising at least part of the extracellular portion of the TGF-beta receptor Type II bound to at least part of a constant region of an immunoglobulin. Preferably, the affinity is in the range of about 10⁻¹¹ to 10⁻¹² M, although the affinity may vary considerably with fragments of different sizes, ranging from 10⁻⁷ to 10⁻¹³ M. In one embodiment, the fragment is about 10-110 amino acids in length and comprises the TGF-beta binding site linked to at least part of a constant region of an immunoglobulin.

If the native amino acid sequence of the extracellular domain of TGF-beta receptor (e.g. amino acids 1 to 160 of SEQ ID NOS: 8 or 9 is used in the fusion proteins, the amino acid sequence resembles that of the entire extracellular portion of the Type II receptor. When smaller TGF beta -receptor Type II portions are employed, they resemble various portions of the extracellular portions of the Type II TGF-beta receptor, so long as they bind TGF-beta with high affinity. Although the sequence of a given "fragment" is based most preferably on the native TGF-beta receptor II extracellular region, the definition also comprises analogs of proteins of the invention which have high affinity for TGF-beta. Such analogs include those made by conservative substitutions of amino acids, as well as those made by mutated cells synthesizing the fragment.

Family members of TGF-beta receptors useful in the uses of compositions in the methods of the invention include any of the naturally-occurring native proteins including allelic, phylogenetic counterparts or other variants thereof, whether naturally-sourced or chemically produced including muteins or mutant proteins, as well as recombinant forms and new, active members of the family. The polypeptides include an amino acid sequence at least 98% or 99% homologous to an amino acid sequence from SEQ ID NOS: 8 or 9. The polypeptide can also include an amino acid sequence essentially the same as an amino acid sequence in SEQ ID NOS: 8 or 9. The polypeptide is at least 5, 10, 20, 50, 100, or 150 amino acids in length and includes at least 5, preferably at least 10, more preferably at least 20, most preferably at least 50, 100, or 150 contiguous amino acids from SEQ ID NOS: 8 or 9.

Polypeptides of the invention include those which arise as a result of the existence of multiple genes, alternative transcription events, alternative RNA splicing events, and alternative translational and posttranslational events. The polypeptide can be made entirely by synthetic means or can be expressed in systems, e.g., cultured cells, which result in substantially the same posttranslational modifications present when the protein is expressed in a native cell, or in systems which result in the omission of posttranslational modifications present when expressed in a native cell.

### B. Isolated Polynucleotide Sequences

Also disclosed are polynucleotides which are derived from polynucleotides that encode, upon expression, the wild-type TGF-beta receptor (e.g. SEQ ID NOS: 1-4, 10 and 12) see also U.S. Patent 5,693,607. A cDNA encoding a TGF-beta receptor Type II molecule (i.e. nucleotides numbered as 832-1311 of SEQ ID NO: 2 or SEQ ID NO: 4 is a complementary DNA (cDNA) sequence encoding for TGF-beta receptor. Other members of the TGF-beta receptor family are presented as wild-type TGF-beta Type I polynucleotides (REF), and wild-type Type III polynucleotide (REF).

Also disclosed are isolated, polynucleotides depicted in SEQ ID NOS: 1-4, 10 which and 12 can be altered to provide for functionally equivalent polynucleotides. A polynucleotide is "functionally equivalent" compared with those of SEQ ID NOS: 1-4, 10 and 12 if it satisfies at least one of the following conditions: (a) the "functional equivalent" is a polynucleotide that hybridizes to any of the foregoing sequences (SEQ ID NOS, 1-4, 10. 12) under standard hybridization conditions. Most preferably, it encodes a TGF-beta receptor protein having the same biological activity as wild-type TGF-beta receptor: and (b) the "functional equivalent" is a polynucleotide that codes on expression for an amino acid sequence encoded by any of the polynucleotides of SEQ ID NOS. 1-4, 10 and 12.

Due to the degeneracy of the nucleotide coding sequences, other polynucleotides may be used to encode TOF-beta receptors or fusions thereof. For TGF-beta Type II receptor, these include all, or portions of DNA encoding SEQ ID NOS.: 8 or 9, which are altered by the substitution of different codons that encode the same amino acid residue within the sequence, thus producing a silent change. Such altered sequences are regarded as equivalents of SEQ ID NOS.: 8 or 9 For example, Phe(f) is coded for by two codons. TTC or TTT, Try (Y) is coded for by TAC or TAT and His (H) is coded for by CAC or CAT. On the other hand, Trp (W) is coded for by a single codon, TGG. Accordingly, it will be appreciated that for a given DNA sequence encoding a particular protein there will be many DNA degenerate sequences that will code for it. These degenerate DNA sequences are considered within the scope of this invention.

Fusion proteins of the invention incorporating the rabbit TGF-beta Type II receptor are shown in SEQ ID NOS: 2 and 8, for the cDNA and deduced amino acids sequences, respectively. Also disclosed herein are fusion proteins incorporating the human TGF-beta Type II receptor shown in SEQ ID NOS: 4 and 9 for the cDNA and deduced amino acid sequences, respectively.

The preferred TGF-beta RII:Fc proteins of the invention include the novel "junction" DNA sequence SEQ ID NO: 10 and amino acid SEQ ID NO: 11. SEQ ID NO: 10 represents the 11 triplet codons on either side of the CTG/GTC junction between the rabbit TGF-betaRII DNA and the DNA of the immunoglobulin. In SEQ ID NO: 10, the TGF-beta sequence ends after the CTG triplet and the immunoglobulin sequence begins at the GTC triplet. The corresponding deduced rabbit amino acid "junction" sequence is represented in SEQ ID NO: 11 in which the end of the TGF-beta receptor sequence is the leucine and the beginning of the Immunoglobulin sequence is the valine immediately thereafter. Also described herein are corresponding "junction" human cDNA and amino acid sequences SEQ ID NOS.: 12 and 13, respectively. In SEQ ID NO: 2, the corresponding junction begins at the triplet starting from nucleotide 1309 and ends at the triplet starting with nucleotide 1312.

It might be that SEQ ID NOS: 10 and 12 are the minimum DNA needed for hybridization under standard conditions to any DNA sequence encoding any TGF-beta receptor:Fc fusion protein. Nevertheless, provided that the whole probe hybridizes to both sides of the junction and both sides of the TGF-beta receptor/constant region junction participate in the hybridization, smaller sequences may exist. Furthermore, persons having ordinary skill in the art will understand that DNA sequences larger than SEQ ID NOS: 10 and 12 will be suitable for hybridization as well. One of ordinary skill in the art can test if a particular probe such as SEQ ID NOS: 10 and 12 are capable of hybridizing on both sides of the junction by labelling the 5' end of either a single strand sense oligonucleotide or a single strand anti-sense oligonucleotide with an appropriately labelled phosphate of ATP using polynucleotide kinase. A sequence of the invention must hybridize to, and thus be labelled by both oligonucleotide probes. It is further understood that the invention encompasses fully degenerate sequences encoding the junction SEQ ID NOS: 10 and 12.

Like most mammalian genes, mammalian TGF-beta receptors are presumably encoded by multi-exon genes. Alternative mRNA constructs which can be attributed to different mRNA splicing events following transcription, and which share large regions of identity or similarity with the cDNAs claimed herein may also be used. The vector containing the TGF-beta RII:Fc cDNA clone , was used to express and purify soluble TGF-beta RII:Fc.

Other mammalian TGF-beta receptor cDNAs may be isolated by using an appropriate human TGF-beta receptor DNA sequence as a probe for screening a particular mammalian cDNA library by cross-species hybridization. Mammalian TGF-beta receptor used in the present invention includes, by way of example, primate, human, murine, canine, feline, bovine, equine and porcine TGF-beta receptor. Mammalian TGF-beta receptors can be obtained by cross species hybridization, using a single stranded cDNA derived from the human TGF-beta receptor DNA sequence as a hybridization probe to isolate TGF-beta receptor cDNAs from mammalian cDNA libraries.

### III. Production of Recombinant Polypeptides

The isolated polypeptides described herein can be produced by any suitable method known in the art. Such methods range from direct protein synthetic methods to constructing a DNA sequence encoding isolated polypeptide sequences and expressing those sequences in a suitable transformed host. For example, cDNA may be obtained by screening a human cDNA library with a labeled DNA fragment encoding the polypeptides of SEQ ID NOS: 8. 9, 11 or 13 and identifying positive clones by autoradiography. Further rounds of plaque purification and hybridization are performed using conventional methods.

In one embodiment of a recombinant method, a DNA sequence is constructed by isolating or synthesizing a DNA sequence encoding a wild-type protein of interest. Optionally, the sequence may be mutagenized by site-specific mutagenesis to provide functional analogs thereof. See, e.g. Zoeller et al., Proc.-Nat Acad. Sci. USA 81:5662-5066 (1984) and United States Patent 4,588,585. Another method of constructing a DNA sequence encoding a polypeptide of interest would be by chemical synthesis using an oligonucleotide synthesizer. Such oligonucleotides may be preferably designed based on the amino acid sequence of the desired polypeptide, and preferably selecting those codons that are favored in the host cell in which the recombinant polypeptide of interest will be produced.

Standard methods may be applied to synthesize an isolated polynucleotide sequence encoding a isolated polypeptide of interest. For example, a complete amino acid sequence may be used to construct a back-translated gene. See Maniatis et al., *supra.* Further, a DNA oligomer containing a nucleotide sequence coding for the particular isolated polypeptide may be synthesized. For example, several small oligonucleotides coding for portions of the desired polypeptide may be synthesized and then ligated. The individual oligonucleotides typically contain 5' or 3' overhangs for complementary assembly.

Once assembled (by synthesis, site-directed mutagenesis or another method), the mutant DNA sequences encoding a particular isolated polypeptide of interest will be inserted into an expression vector and operatively linked to an expression control sequence appropriate for expression of the protein in a desired host. Proper assembly may be confirmed by nucleotide sequencing, restriction mapping, and expression of a biologically active polypeptide in a suitable host. As is well known in the art, in order to obtain high expression levels of a transfected gene in a host, the gene must be operatively linked to transcriptional and translational expression control sequences that are functional in the chosen expression host.

### A. Expression of Recombinant TGF-beta Receptor

Recombinant expression vectors are preferably used to amplify or express DNA encoding TGF-beta receptor fusions Recombinant expression vectors are replicable DNA constructs which have synthetic or cDNA-derived DNA fragments encoding TGF-beta receptor fusions or bioequivalent analogs operatively linked to suitable transcriptional or translational regulatory elements derived from mammalian, microbial, viral or insect genes. A transcriptional unit generally comprises an assembly of (1) a genetic element or elements having a regulatory role in gene expression, for example, transcriptional promoters or enhancers, (2) a structural or coding sequence which is transcribed into mRNA and translated into protein, and (3) appropriate transcription and translation initiation and termination sequences, as described in detail below. Such regulatory elements may include an operator sequence to control transcription. The ability to replicate in a host, usually conferred by an origin of replication, and a selection gene to facilitate recognition of transformants may additionally be incorporated. DNA regions are operatively linked when they are functionally related to each other. For example, DNA for a signal peptide (secretory leader) is operatively linked to DNA for a polypeptide if it is expressed as a precursor which participates in the secretion of the polypeptide: a promoter is operatively linked to a coding sequence if it controls the transcription of the sequence; or a ribosome binding site is operatively linked to a coding sequence if it is positioned so as to permit translation. Generally, operatively linked means contiguous and, in the case of secretory leaders, contiguous and in reading frame. Structural elements intended for use in yeast expression systems preferably include a leader sequence enabling extracellular secretion of translated protein by a host cell. Alternatively, where recombinant protein is expressed without a leader or transport sequence, it may include an N-terminal methionine residue. This residue may optionally be subsequently cleaved from the expressed recombinant protein to provide a final product.

DNA sequences encoding mammalian TGF-beta receptors which are to be expressed as a fusion in microorganism may preferably contain introns that could prematurely terminate transcription of DNA into mRNA since such premature termination of transcription may be desirable, for example, where it would result in mutants having advantageous C-terminal truncations, for example, deletion of a transmembrane region to yield a soluble receptor not bound to the cell membrane. Due to code degeneracy, there can be considerable variation in nucleotide sequences encoding the same amino acid sequence. As discussed above, other embodiments include sequences capable of hybridizing to the sequences of the provided cDNA under standard conditions (50 degrees C., 2 x SSC) and other sequences hybridizing or degenerate to those which encode biologically active proteins of the invention.

The choice of expression control sequence and expression vector will depend upon the choice of host. A wide variety of expression host/vector combinations may be employed. Useful expression vectors for eukaryotic hosts, include, for example, vectors comprising expression control sequences from SV40, bovine papilloma virus, adenovirus and cytomegalovirus. Useful expression vectors for bacterial hosts include known bacterial plasmids, such as plasmids from *Esherichia. coli,* including pCR1, pBR322, pMB9 and their derivatives, wider host range plasmids, such as M 13 and filamentous single-stranded DNA phages. Preferred *E. coli* vectors include pL vectors containing the lambda phage pL promoter (U.S. Patent 4,874,702), pET vectors containing the T7 polymerase promoter (Studier et al., Methods in Enzymology 185: 60-89, 1990 1) and the pSP72 vector (Kaelin et al., *supra*). Useful expression vectors for yeast cells, for example, include the 2 µ and centromere plasmids.

In addition, any of a wide variety of expression control sequences may be used in these vectors. Such useful expression control sequences include the expression controi sequences associated with structural genes of the foregoing expression vectors. Examples of useful expression control sequences include, for example, the early and late promoters of SV40 or adenovirus. the *lac* system, the *trp* system, the *TAC* or *TRC* system, the major operator and promoter regions of phage lambda, for example pL. the control regions of fd coat protein, the promoter for 3-phosphoglycerate kinase or other glycolytic enzymes, the promoters of acid phosphatase, e.g., Pho5, the promoters of the yeast *alpha*-mating system and other sequences known to control the expression of genes of prokaryotic or eukaryotic cells and their viruses, and various combinations thereof.

Recombinant TGF-beta receptor DNA or recombinant TGF-beta R:Fc DNA is expressed or amplified in a recombinant expression system comprising a substantially homogeneous monoculture of a suitable host which have stably integrated (by transformation or transfection) a recombinant transcriptional unit into chromosomal DNA or carry the recombinant transcriptional unit as a component of a resident plasmid. Generally, cells constituting the system are the progeny of a single ancestral transformant. Recombinant expression systems as defined herein will express heterologous protein upon induction of the regulatory elements linked to the DNA sequence or synthetic gene to be expressed.

Suitable host cells for expression of mammalian TGF-beta receptor and its fusion proteins include prokaryotes, yeast or higher eukaryotic cells under the control of appropriate promoters. Prokaryotes include gram negative or gram positive organisms, for example *E. coli* or bacilli. Higher eukaryotic cells include established cell lines of mammalian origin as described below. Cell-free translation systems could also be employed to produce mammalian TGF-beta receptor and TGF-beta R:Fc using RNAs derived from the DNA constructs of the present invention. Appropriate cloning and expression vectors for use with bacterial, fungal, yeast, and mammalian cellular hosts are described by Pouwels et al. (Cloning Vectors: A Laboratory Manual. Elsevier, N.Y., 1985).

Recombinant proteins of the invention may also be expressed in yeast hosts, preferably from the *Saccharomyces* species, such as *S. cerevisiae.* Yeast of other genera, such as Pichia or Kluyveromyces may also be employed. Yeast vectors will generally contain an origin of replication from the 2 mu yeast plasmid or an autonomously replicating sequence (ARS), promoter. DNA encoding proteins of the invention and DNA sequences for polyadenylation and transcription termination and a selection gene. Preferably, yeast vectors will include an origin of replication and selectable marker permitting transformation of both yeast and *E. coli.,* e.g., the ampicillin resistance gene of *E. coli.* and *S. cerevisiae* TRP1 or URA3 gene, which provides a selection marker for a mutant strain of yeast lacking the ability to grow in tryptophan, and a promoter derived from a highly expressed yeast gene to induce transcription of a structural sequence downstream. The presence of the TRP1 or URA3 lesion in the yeast host cell genome then provides an effective environment for detecting transformation by growth in the absence of tryptophan or uracil.

Suitable promoter sequences in yeast vectors include the promoters for metallothionein, 3-phosphoglycerate kinase (Hitzeman et al., J. Biol. Chem. 255:2073, 1980) or other glycolytic enzymes (Hess et al., J. Adv. Enzyme Reg. 7:149, 1968; and Holland et al., Biochem. 7:4900, 1978), such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase. Suitable vectors and promoters for use in yeast expression are further described in R. Hitzeman et al., EPA 73,657. Preferred yeast vectors can be assembled using DNA sequences from pUC18 for selection and replication in E. coli. (Amp' gene and origin of replication) and yeast DNA sequences including a glucose-repressible ADH2 promoter and *alpha*-factor secretion leader. The ADH2 promoter has been described by Russell et al. (J. Biol. Chem. 258:2674, 1982) and Beier et al. (Nature 300:724, 1982). The yeast *alpha* -factor leader, which directs secretion of heterologous proteins, can be inserted between the promoter and the structural gene to be expressed. See, e.g., Kurjan et al., Cell 30:933, 1982; and Bitter et al.. Proc. Natl. Acad. Sci. U.S.A. 81:5330. 1984. The leader sequence may be modified to contain, near its 3' end, one or more useful restriction sites to facilitate fusion of the leader sequence to foreign genes.

Suitable yeast transformation protocols are known to those of skill in the art: an exemplary echnique is described by Hinnen et al., Proc. Natl. Acad. Sci. U.S.A. 75:1929, 1978 as described by Sherman et al., Laboratory Course Manual for Methods in Yeast Genetics. Cold Spring Harbor Laboratory, Cold Spring Harbor, N.Y., 1986.

Various mammalian or insect cell culture systems are also advantageously employed to express recombinant protein. Expression of recombinant proteins in mammalian cells is particularly preferred because such proteins are generally correctly folded, appropriately modified and completely functional. Examples of suitable mammalian host cell lines include the COS-7 lines of monkey kidney cells, described by Gluzman (Cell 23:175, 1981), and other cell lines capable of expressing an appropriate vector including, for example, L cells, C127, 3T3, Chinese hamster ovary (CHO). HeLa and BHK cell lines. Mammalian expression vectors may comprise nontranscribed elements such as an origin of replication, a suitable promoter and enhancer linked to the gene to be expressed, and other 5' or 3' flanking nontranscribed sequences, and 5' or 3' nontranslated sequences.such as necessary ribosome binding sites, a polyadenylation site, splice donor and acceptor sites, and transcriptional termination sequences. Baculovirus systems for production of heterologous proteins in insect cells are reviewed by Luckow and Summers, Bio/Technology 6:47 (1988).

Recombinant expression vectors comprising cDNAs are stably integrated into a host cell's DNA. Elevated levels of expression product is achieved by selecting for cell lines having amplified numbers of vector DNA. Cell lines having amplified numbers of vector DNA are selected, for example, by transforming a host cell with a vector comprising a DNA sequence which encodes an enzyme which is inhibited by a known drug. The vector may also comprise a DNA sequence which encodes a desired protein. Alternatively, the host cell may be co-transformed with a second vector which comprises the DNA sequence which encodes the desired protein. The transformed or co-transformed host cells are then cultured in increasing concentrations of the known drug, thereby selecting for drug-resistant cells. Such drug-resistant cells survive in increased concentrations of the toxic drug by over-production of the enzyme which is inhibited by the drug, frequently as a result of amplification of the gene encoding the enzyme. Where drug resistance is caused by an increase in the copy number of the vector DNA encoding the inhibitable enzyme, there is a concomitant co-amplification of the vector DNA encoding the desired protein in the host cell's DNA.

A preferred system for such co-amplification uses the gene for dihydrofolate reductase (DHFR), which can be inhibited by the drug methotrexate (MTX). To achieve co-amplification, a host cell which lacks an active gene encoding DHFR is either transformed with a vector which comprises DNA sequence encoding DHFR and a desired protein, or is co-transformed with a vector comprising a DNA sequence encoding DHFR and a vector comprising a DNA sequence encoding the desired protein. The transformed or co-transformed host cells are cultured in media containing increasing levels of MTX, and those cells lines which survive are selected.

A particularly preferred co-amplification system uses the gene for glutamine synthetase (GS), which is responsible for the synthesis of glutamate and ammonia using the hydrolysis of ATP to ADP and phosphate to drive the reaction. GS is subject to inhibition by a variety of inhibitors, for example methionine sulphoximine (MSX). Thus, proteins of the invention can be expressed in high concentrations by co-amplifying cells transformed with a vector comprising the DNA sequence for GS and a desired protein, or co-transformed with a vector comprising a DNA sequence encoding GS and a vector comprising a DNA sequence encoding the desired protein, culturing the host cells in media containing increasing levels of MSX and selecting for surviving cells. The GS co-amplification system, appropriate recombinant expression vectors and cells lines, are described in the following PCT applications: WO 87/04462, WO 89/01036, WO 89/10404 and WO 86/05807.

Recombinant proteins are preferably expressed by co-amplification of DHFR or GS in a mammalian host cell, such as Chinese Hamster Ovary (CHO) cells, or alternatively in a murine myeloma cell line, such as SP2/0-Agl4 or NS0 or a rat myeloma cell line, such as YB2/3.0-Ag20. disclosed in PCT applications WO/89/10404 and WO 86/05807.

It should be understood that not all vectors and expression control sequences will function equally well to express a given isolated polypeptide. Neither will all hosts function equally well with the same expression system. However, one of skill in the art may make a selection among these vectors, expression control systems and hosts without undue experimentation.

### B. Purification of Recombinant TGF-betaRII:Fc

The proteins produced by a transformed host can be purified according to any suitable method. Such standard methods include chromatography (e.g., ion exchange, affinity and sizing column chromatography), centrifugation, differential solubility, or by any other standard technique for protein purification. Affinity tags such as hexahistidine, maltose binding domain, influenza coat sequence and glutathione-S-transferase can be attached to the protein to allow easy purification by passage over an appropriate affinity column. Isolated proteins can also be physically characterized using such techniques as proteolysis, nuclear magnetic resonance and x-ray crystallography.

For example, supernatants from systems which secrete recombinant protein into culture media can be first concentrated using a commercially available protein concentration filter, for example, an Amicon or Millipore Pellicon ultrafiltration unit. Following the concentration step, the concentrate can be applied to a suitable purification matrix. For example, a suitable affinity matrix can comprise a TGF-beta or lectin or antibody molecule or Protein A bound to a suitable support. Alternatively, an anion exchange resin can be employed, for example, a matrix or substrate having pendant diethylaminoethyl (DEAE) groups. The matrices can be acrylamide, agarose, dextran, cellulose or other types commonly employed in protein purification. Alternatively, a cation exchange step can be employed. Suitable cation exchangers include various insoluble matrices comprising sulfopropyl or carboxymethyl groups. Sulfopropyl groups are preferred. Finally, one or more reversed-phase high performance liquid chromatography (RP-HPLC) steps employing hydrophobic RP-HPLC media, e.g., silica gel having pendant methyl or other aliphatic groups, can be employed to further purify a TGF-betaRII:Fc composition. Some or all of the foregoing purification steps, in various combinations, can also be employed to provide a homogeneous recombinant protein.

Recombinant protein produced in bacterial culture is usually isolated by initial extraction from cell pellets, followed by one or more concentration, salting-out, aqueous ion exchange or size exclusion chromatography steps. Finally, high performance liquid chromatography (HPLC) can be employed for final purification steps. Microbial cells employed in expression of recombinant mammalian TGF-beta receptor can be disrupted by any convenient method, including freeze-thaw cycling, sonication, mechanical disruption, or use of cell lysing agents.

Fermentation of yeast which express mammalian TGF-beta receptor fusion as a secreted protein greatly simplifies purification. Secreted recombinant protein resulting from a large-scale fermentation can be purified by methods analogous to those disclosed by Urdal et al. (J. Chromatog. 296:171, 1984). This reference describes two sequential, reversed-phase HPLC steps for purification of recombinant human GM-CSF on a preparative HPLC column.

### C. Production of Fragments and Analogs

Fragments of an isolated protein (e.g., fragments of SEQ ID NO: 8) can also be produced efficiently by recombinant methods, by proteolytic digestion, or by chemical synthesis, using methods known to those of skill in the art. In recombinant methods, internal or terminal fragments of a polypeptide can be generated by removing one or more nucleotides from one end (for a terminal fragment) or both ends (for an internal fragment) of a DNA sequence which encodes for the isolated polypeptide. Expression of the mutagenized DNA produces polypeptide fragments. Digestion with "end nibbling" endonucleases can also generate DNAs which encode an array of fragments. DNAs which encode fragments of a protein can also be generated by random shearing, restriction digestion or a combination or both.

TGF-beta receptor fragments can be generated directly from intact TGF-beta receptor proteins. Peptides can be specifically cleaved by proteolytic enzymes, including, but not limited to plasmin, thrombin, trypsin, chymotrypsin or pepsin. Each of these enzymes is specific for the type of peptide bond it attacks. Trypsin catalyzes the hydrolysis of peptide bonds in which the carbonyl group is from a basic amino acid, usually arginine or lysine. Pepsin and chymotrypsin catalyse the hydrolysis of peptide bonds from aromatic amino acids, such as tryptophan, tyrosine and phenylalanine. Alternate sets of cleaved protein fragments are generated by preventing cleavage at a site which is suceptible to a proteolytic enzyme. For instance, reaction of the ε-amino acid groups of lysine with ethyltrifluorothioacetate in mildly basic solution yields blocked amino acid residues whose adjacent peptide bond is no longer susceptible to hydrolysis by trypsin. Proteins can be modified to create peptide linkages that are susceptible to proteolytic enzymes. For instance, alkylation of cysteine residues with β-halo ethylamines yields peptide linkages that are hydrolyzed by trypsin (Lindley, Nature 178: 647 (1956)). In addition, chemical reagents that cleave peptide chains at specific residues can be used. For example, cyanogen bromide cleaves peptides at methionine residues (Gross and Witkip, J. Am. Chem. Soc., 83: 1510 (1961)). Thus, by treating proteins with various combinations of modifiers, proteolytic enzymes and/or chemical reagents, the proteins may be divided into fragments of a desired length with no overlap of the fragments, or divided into overlapping fragments of a desired length.

Fragments can also be chemically synthesized using techniques known in the art such as the Merrifield solid phase F moc or t-Boc chemistry. Merrifield, Recent Progress in Hormone Research 23: 451 (1967**)**

### D. Production of Altered DNA and Peptide Sequences: Random Methods

Amino acid sequence variants of a protein (such as variants of SEQ ID NO.: 8) can be prepared by random mutagenesis of DNA which encodes the protein or a particular portion thereof. Useful methods include PCR mutagenesis and saturation mutagenesis. A library of random amino acid sequence variants can also be generated by the synthesis of a set of degenerate oligonucleotide sequences. Methods of generating amino acid sequence variants of a given protein using altered DNA and peptides are well-known in the art. The following examples of such methods are not intended to limit the scope of the present invention, but merely serve to illustrate representative techniques. Persons having ordinary skill in the art will recognize that other methods are also useful in this regard.

PCR Mutagenesis: Briefly, *Taq* polymerase (or another polymerase) is used to introduce random mutations into a cloned fragment of DNA (See Leung et al., Technique 1: 11-15 (1989)). PCR conditions are chosen so that the fidelity of DNA synthesis is reduced by Taq DNA polymers using, for instance, a dGTP/dATP ratio of five and adding Mn⁺² to the PCR reaction. The pool of amplified DNA fragments is inserted into appropriate cloning vectors to provide random mutant libraries.

Saturation Mutagenesis: One method is described generally in Mayers et al., Science. 229: 242 (1989). Briefly, the technique includes generation of mutations by chemical treatment or irradiation of single stranded DNA *in vitro,* and synthesis of a cDNA strand. The mutation frequency is modulated by the severity of the treatment and essentially all possible base substitutions can be obtained.

Degenerate Oligonucleotide Mutagenesis: A library of homologous peptides can be generated from a set of degenerate oligonucleotide sequences. Chemical synthesis of degenerate sequences can by performed in an automatic DNA synthesizer, and the synthetic genes then ligated into an appropriate expression vector. See Itakura et al., Recombinant DNA, Proc. 3rd Cleveland Symposium on Macromolecules, pp. 273-289 (A.G. Walton, ed.), Elsevier, Amsterdam, 1981.

### E. Production of Altered DNA and Peptide Sequences: Directed Methods

Non-random, or directed, mutagenesis provides specific sequences or mutations in specific portions of a polynucleotide sequence that encodes an isolated polypeptide, to provide variants which include deletions, insertions or substitutions of residues of the known amino acid sequence of the isolated polypeptide. The mutation sites may be modified individually or in series, for instance by: (1) substituting first with conserved amino acids and then with more radical choices depending on the results achieved; (2) deleting the target residue; or (3) inserting residues of the same or a different class adjacent to the located site, or combinations of options 1-3.

Alanine scanning Mutagenesis: This method locates those residues or regions of a desired protein that are preferred locations for mutagenesis. See Cunningham and Wells, Science 244: 1081-1085 (1989). In alanine screening, a residue or group of target residues are selected and replaced by alanine or polyalanine. This replacement can affect the interaction of the amino acid with neighboring amino acids and/or with the surrounding aqueous or membrane environment. Those having functional sensitivity to the substitutions are then refined by introducing further or other variants at, or for, the sites of substitution.

Oligonucleotide-Mediated Mutagenesis: One version of this method may be used to prepare substitution, deletion, and insertion variants of DNA. See Adelman et al., DNA 2:183 (1983). Briefly, the desired DNA is altered by hybridizing an oligonucleotide primer encoding a DNA mutation to a DNA template which typically is the single stranded form of a plasmid or phage containing the unaltered or wild-type DNA sequence template of the desired protein (e.g., the protein). After hybridization, a DNA polymerase is used to make the second and complementary strand of DNA of the template that will incorporate the oligonucleotide primer, and will code for the selected alteration in the desired DNA sequence. Generally, oligonucleotides of at least 25 nucleotides in length are used. An optimal oligonucleotide will have 12 to 15 nucleotides that are completely complementary to the template on either side of the mutation. This ensures that the oligonucleotide will hybridize properly to the single-stranded DNA template molecule.

Cassette Mutagenesis: This method (Wells et al., Gene 34: 315 (1985)) requires a plasmid or other vector that contains the protein subunit DNA to be mutated. The codon(s) in the protein subunit DNA are identified and there is inserted a unique restriction endonuclease site on each side of the identified mutation site(s), using the above-described oligonucleotide-directed mutagenesis method. The plasmid is then cut at these sites to linearize it. A double-stranded oligonucleotide encoding the sequence of the DNA between the restriction sites but containing the desired mutation(s) is synthesized using standard procedures. The two strands are synthesized separately and then hybridized together using standard methods. This double-stranded oligonucleotide is the "cassette" and it has 3' and 5' ends that are compatible with the ends of the linearized plasmid so that it can be directly ligated therein. The plasmid now contains the mutated desired protein subunit DNA sequence.

Combinatorial Mutagenesis: In one version of this method (Ladner et al., W0 88/06630), the amino acid sequences for a group of homologs or other related proteins are aligned, preferably to promote the highest homology possible. All of the amino acids which appear at a given position of the aligned sequences can be selected to create a degenerate set of combinatorial sequences. The variegated library is generated by combinatorial mutagenesis at the nucleic acid level, and is encoded by a variegated gene library. For instance, a mixture of synthetic oligonucleotides can be enzymatically ligated into the gene sequence such that the degenerate set of potential sequences are expressible as individual peptides, or alternatively, as a set of proteins containing the entire set of degenerate sequences.

### IV. Other Variants of Isolated Polypeptides

Derivatives of proteins of the invention also include various structural forms of the primary protein which retain biological activity. Due to the presence of ionizable amino and carboxyl groups, for example, a TGF-beta receptor fusion protein may be in the form of acidic or basic salts, or may be in neutral form. Individual amino acid residues may also be modified by oxidation or reduction. The primary amino acid structure (e.g., of the TGF-beta RII moiety) may be modified by forming covalent or aggregative conjugates with other chemical moieties, such as glycosyl groups, lipids, phosphate, acetyl groups and the like, or by creating amino acid sequence mutants.

Covalent derivatives of the TGF-beta receptor may prepared by linking particular functional groups to TGF-beta receptor amino acid side chains or at the N- or C-termini. Other derivatives of TGF-beta RII:Fc include covalent or aggregative conjugates of TGF-beta RII or its fragments with other proteins or polypeptides, such as by synthesis in recombinant culture as additional N-terminal or C-terminal fusions. For example, the conjugated peptide may be a signal (or leader) polypeptide sequence at the N-terminal region of the protein which co-translationally or post-translationally directs transfer of the protein from its site of synthesis to its site of function inside or outside of the cell membrane or wall (e.g., the yeast alpha -factor leader). TGF-beta receptor proteins can comprise peptides added to facilitate purification or identification of TGF-beta receptor (e.g., poly-His). The amino acid sequence of TGF-beta receptor can also be linked to the peptide Asp-Tyr-Lys-Asp-Asp-Asp-Asp-Lys (DYKDDDDK) (Hopp et al., Bio/Technology 6:1204,1988.) The latter sequence is highly antigenic and provides an epitope reversibly bound by a specific monoclonal antibody, enabling rapid assay and facile purification of expressed recombinant protein. This sequence is also specifically cleaved by bovine mucosal enterokinase at the residue immediately following the Asp-Lys pairing. Fusion proteins capped with this peptide may also be resistant to intracellular degradation in E. coli.

Polyvalent forms of TGF-beta receptor are useful since they possess multiple TGF-beta receptor binding sites for TGF-beta ligand. For example, a bivalent soluble TGF-beta receptor may consist of two tandem repeats of amino acids numbered 1-160 of SEQ. ID. NO.: 8 or 9 separated by a linker region, the repeats bound to the immunoglobulin constant domain. Alternate polyvalent forms may also be constructed, for example, by chemically coupling TGF-beta receptor Fc fusions to any clinically acceptable carrier molecule, a polymer selected from the group consisting of Ficoll, polyethylene glycol or dextran using conventional coupling techniques. Alternatively, TGF-beta receptor fusion may be chemically coupled to biotin, and the biotin-TGF-beta receptor Fc conjugate then allowed to bind to avidin, resulting in tetravalent avidin/biotin/TGF-beta receptor molecules. TGF-beta receptor Fc fusions may also be covalently coupled to dinitrophenol (DNP) or trinitrophenol (TNP) and the resulting conjugate precipitated with anti-DNP or anti-TNP-IgM, to form decameric conjugates with a valency of 10 for TGF-beta receptor binding sites.

Functional mutant analogs of the TGF-betaRII:Fc fusion having inactivated N-glycosylation sites can be produced by oligonucleotide synthesis and ligation or by site-specific mutagenesis techniques. These analog proteins can be produced in a homogeneous, reduced-carbohydrate form in good yield using yeast expression systems. N-glycosylation sites in eukaryotic proteins are characterized by the amino acid triplet Asn-Al-Z, where Al is any amino acid except Pro, and Z is Ser or Thr. In this sequence. Asn provides a side chain amino group for covalent attachment of carbohydrate. Such a site can be eliminated by substituting another amino acid for Asn or for residue Z, deleting Asn or Z, or inserting a non-Z amino acid between A 1 and Z, or an amino acid other than Asn between Asn and Al. TGF-beta receptor derivatives may also be obtained by mutations of TGF-beta receptor or its subunits. Bioequivalent analogs of TGF-beta receptor proteins may be constructed by, for example, making various substitutions of residues or sequences or deleting terminal or internal residues or sequences not needed for biological activity.

Other analogs include TGF-beta RII:Fc protein or its biologically active fragments whose sequences differ from SEQ ID NOS: 2 or 4 by one or more conservative amino acid substitutions or by one or more non conservative amino acid substitutions, or by deletions or insertions which do not abolish the isolated protein's biological activity. Conservative substitutions typically include the substitution of one amino acid for another with similar characteristics such as substitutions within the following groups: valine, alanine and glycine; leucine and isoleucine: aspartic acid and glutamic acid; asparagine and glutamine; serine and threonine; lysine and arginine; and phenylalanine and tyrosine. The non-polar hydrophobic amino acids include alanine, leucine, isoleucine, valine, proline, phenylalanine, tryptophan and methionine. The polar neutral amino acids include glycine, serine, threonine, cysteine, tyrosine, asparagine and glutamine. The positively charged (basic) amino acids include arginine, lysine and histidine. The negatively charged (acidic) amino acids include aspartic acid and glutamic acid. Other conservative substitutions can be readily known by workers of ordinary skill. For example, for the amino acid alanine, a conservative substitution can be taken from any one of D-alanine, glycine, beta-alanine, L-cysteine and D-cysteine. For lysine, a replacement can be any one of D-lysine, arginine, D-arginine, homo-arginine, methionine, D-methionine, ornithine, or D-ornithine.

Generally, substitutions that may be expected to induce changes in the functional properties of isolated polypeptides are those in which: (i) a polar residue, e.g., serine or threonine, is substituted for (or by) a hydrophobic residue, e.g., leucine, isoleucine, phenylalanine, or alanine; (ii) a cysteine residue is substituted for (or by) any other residue: (iii) a residue having an electropositive side chain, e.g., lysine, arginine or histidine, is substituted for (or by) a residue having an electronegative side chain, e.g., glutamic acid or aspartic acid; or (iv) a residue having a bulky side chain, e.g., phenylalanine, is substituted for (or by) one not having such a side chain, e.g., glycine.

Also disclosed are isolated molecules that are: allelic variants, natural mutants, induced mutants, proteins encoded by DNA that hybridize under high or low stringency conditions to a nucleic acid which encodes a polypeptide such as SEQ. ID. NOS. 8 or 9 and polypeptides specifically bound by antisera to peptides, especially by antisera to an active site or binding site of. All variants described herein are expected to: (i) retain the biological function of the original protein

### V. Utilities

This invention provides compositions and uses of compositions for administering to an individual with a medical condition a sufficient amount of TGF-beta receptor fusion protein, such as TGF-beta RII:Fc, to reduce TGF-beta activity in the individual. This invention further provides compositions and uses of compositions for delivering TGF-beta RII:Fc to a site where TGF-beta is in excess, such as in disease states characterized by fibroproliferation and immunosuppression such as is associated with infectious disease.

While not wishing to be bound by any particular theory, it appears that the TGF-beta RII:Fc regulates TGF-beta activity by competing for TGF-beta with cell-surface receptors. It is further believed that it inactivates TGF-beta by removing it from the free pool of TGF-beta available to interact with cell surface receptors. Depending on the pharmacologic properties of clearance, the TGF beta -RII:Fc/TGF-beta complex is removed from the site of TGF-beta. This complexing with excess TGF-beta reduces the amount of free TGF-beta With less TGF-beta available to complex with cell receptors, TGF-beta induced fibroproliferation slows down, resulting in stasis of the disease state.

The administration of proteins of the invention or fragments of the present invention may be used in fibroproliferative disorders. As mentioned above, animal models of glomerulonephritis have shown good results with anti-TGF-beta antibodies blocking excess TGF-beta. These antibodies will be difficult to deliver because they have a high molecular weight and they may result in severe allergic reactions when they are derived from other species. Thus, it would be preferable to administer a lower molecular weight, native protein or close analog, such as TGF-beta RII:Fc, in glomerulonephritis. Kidney disorders associated with TGF-beta excess include, but are not limited to, mesangial proliferative glomerulonephritis, crescentic glomerulonephritis, diabetic nephropathy, renal interstitial fibrosis, renal fibrosis in transplant patients receiving cyclosporin, and HIV-associated nephropathy. These conditions are associated with overproduction of fibrous tissue formation which administration of TGF-beta RII:Fc should suppress.

As TGF-beta RII by itself is not known to have an effect aside from capturing TGF-beta TGF-beta RII:Fc may safely be administered during or at the end of retinal reattachment surgery, which is the most common cause of proliferative vitreoretinopathy (PVR) (Connor et al. (1989) J. Clin. Invest. 83:1661-1666). Another important fibroproliferative condition is rheumatoid arthritis (RA), which is also associated with excess TGF-beta production. Data indicate that blocking TGF-beta at any time in the development or chronic stages of RA may help stop the progressive deterioration of the joint and bone. Hence, fusions of the present invention may be administered to patients with early joint pain and to patients with prolonged joint pain and deteriorated joints. The current theory is that joint deterioration in RA is due to an overproduction of TGF-beta. Excess TGF-beta has been measured in joints after test animals are injected with streptococcal cell walls, whose presence is believed to cause rheumatoid arthritis (RA). Because anti-TGF-beta antibody blocks arthritic changes in this model, it is believed that fusions of the invention may also have a positive effect. Other conditions associated with excess TGF-beta levels include idiopathic pulmonary fibrosis and myelofibrosis. To complex with excess TGF-beta and to slow the development of excess fibrous tissue, proteins of the invention are intended for administration in these conditions.

The proteins of the present invention may be used to treat collagen vascular disorders that are associated with overproduction of TGF-beta. It is currently believed that there is an overproduction of TGF-beta in collagen vascular disorders, such as progressive systemic sclerosis (PSS), polymyositis, scleroderma, dermatomyositis, eosinophilic fascitis, and morphea. Collagen vascular disorders may also be associated with the occurrence of Raynaud's syndrome. Among other effects, TGF-beta overproduction may also be involved in interstitial pulmonary fibrosis, an end-stage lung disorder which is associated with autoimmune disorders such as systemic lupus erythematosus (SLE) and scleroderma (Deguchi. (1992) Ann. Rheum. Dis. 51:362-65); or it may be caused by chemical contact, allergies to dust and hay fever. A therapeutically effective amount of the proteins of this invention may be administered to neutralize the biologic activity of TGF-beta , which in turn would prevent unwanted fibrosis.

Proteins of the present invention also may be used in preventing overproduction of scarring in patients who are known to form keloids or hypertrophic scars. For example TGF-beta RII:Fc may be administered to prevent scarring or overproduction of scarring during healing of various types of wounds including surgical incisions and traumatic lacerations. The TGF-beta RII:Fc fusion may be applied to skin wounds before they are closed to help in healing without scar formation. Proteins of the invention may be placed in surgical abdominal wounds to help prevent adhesion formation which occurs all too commonly after that type of surgery. The present invention provides for the local administration of sufficient fusion to complex with local TGF-beta overproduction and prevent overproduction of healing processes.

TGF-beta excess also has been reported in nasal polyposis, a condition characterized by multiple polyps (Ohno et al. (1992) J. Clin. Invest. 89: 1662-68). Fusion proteins can help lower TGF-beta levels and slow the hyperproliferation that results in polyps. For example, TGF-beta RII:Fc can be administered after polyp surgery to prevent overproduction of scarring and recurrence of polyps and can also be administered to inhibit polyp formation in the intestine.

Proteins of the invention may also be administered following coronary angioplasty, preferably placed along the inside of the affected arteries. According to Karas et al., ((1991) Clin. Cardiol. 14:791-801) restenosis or scarring and reclosing of arteries following coronary angioplasty is seen in approximately one-third of patients operated on. Because the fibrous network which ultimately develops into a scar normally accumulates rapidly, early administration of, for instance, TGF-beta RII:Fc would reduce excess TGF-beta in this area and slow excessive proliferation of connective tissue and restenosis.

TGF-beta excess has also been observed in cardiac fibrosis after infarction and in hypertensive vasculopathy. To aid in proper healing without excess scar or fibrous tissue formation, proteins of the invention can be administered in these conditions. TGF-beta excess also has been observed in the tissues of patients receiving radiation therapy. Such tissue is characterized by excess connective tissue development, epithelial thinning and blood vessel occlusion associated with overgrowth of endothelial cells. Administration of an TGF-beta RII:Fc will complex with the excess TGF-beta and will contribute to healing without excessive fibrosis.

### VI. Formulation, Administration and Dosage

The formulation, method of administration and dosage of proteins of the invention will depend upon the disorder to be treated, and the medical history of the patient. These factors are readily determinable in the course of therapy. Suitable patients with conditions caused by an excess of TGF-beta can be identified by laboratory tests, medical history and physical findings. TGF-beta excess can be determined directly by immunoassay of the patient's serum or of the affected tissue. Excess TGF-beta can also be determined by bioassays such as the cell proliferation assay described in Kekow et al., (1990) Proc. Natl. Acad. Sci. U.S.A. 87: 8321-25. Excess TGF-beta also can be determined indirectly by measuring the level of TGF-beta mRNA (for example, in the polymerase chain reaction of Kekow et al.).

The medical history may reveal facts which support a diagnosis of fibroproliferative disorder, collagen vascular disorder, immunosuppression, or of potential for problematic wound healing, as in peritoneal adhesions following surgery., or restenosis of blood vessels after coronary angioplasty. Conditions which are identified as being associated with high levels of TGF-beta and/or proliferation of fibrous tissue are considered to cause TGF-beta excess. Patients may have a wide spectrum of physical findings which are indicative of such disorders. Skin biopsies have been used to test TGF-beta in patients with systemic sclerosis. Swollen, hot joints are seen in arthritis. In accordance with the uses of the compositions in the method of the present invention, the formulation comprises proteins of the invention in an administrable form. The uses of the compositions in the method of the present invention provide for formulating proteins of the invention in modes which are readily apparent to those skilled in the art. Preferably, the proteins of the invention are dissolved in physiologically compatible carriers.

Physiologically compatible carriers for proteins of the invention include intravenous solutions, such as normal saline, serum albumin, 5% dextrose, plasma preparations, other protein-containing solutions and TPN solutions. The preferred carrier for parenteral administration is a sterile, isotonic aqueous solution, such as saline or 5% dextrose. Even more preferred is normal saline with human serum albumin. For use in enhancing the immune response to vaccines, proteins of the invention may be mixed with the vaccine formulation.

Depending on the mode of administration, the proteins of the invention may be in the form of liquid or semi-solid dosage preparations, such as for example, liquids, suspensions or the like. Alternatively, a solution may be placed into an implant, such as an osmotic pump, for the slow release over an extended period of time. Alternatively, proteins of the invention may be provided in sustained release carrier formulations such as semi-permeable polymer carriers in the form of suppositories or microcapsules. See, for instance, U.S. Pat. No. 3,773,919 for microcapsular sustained release matrices including polylactides; Sidmon et al., Biopolymers 22 (1), 547-556 (1983) for copolymers of L-glutamic acid and gamma -ethyl-L-glutamate; Langer et al., J. Biomed. Res. 15, 167-277 (1981) for poly(2-hydroxyethylmethacrylate) or the like.

The mode of administration delivers proteins of the invention to the individual in a safe, physiologically effective manner. Proteins of the invention may be given by intraocular, intranasal, subcutaneous, intravenous, intramuscular, intradermal, intraperitoneal, intraarticular, enteral or other conventional routes of administration. In a preferred embodiment, the proteins of the invention of the invention is administered locally to the affected tissue sites by bolus injection or perfusion. For example, for PVR, the preferred mode of administration is a single intraocular injection. Local administration is also preferred in peritoneal wounds to avoid adhesion formation and in other wounds to encourage healing with no keloids or visible scars. For nasal polyposis, nasal drops are preferred. Local and systemic administration are equally preferred in lung fibrosis (parenteral injection or nasal spray or drops). Rheumatoid arthritis (RA) can be treated by intraarticular or systemic administration.

Systemic administration is the preferred mode of administration in glomerulonephritis and in widespread skin disorders (such as progressive systemic sclerosis, diffuse fascitis, and generalized morphea). Systemic administration also is preferred when proteins of the invention is used to enhance vaccine response, proteins of the invention can be administered with the vaccine by subcutaneous, intramuscular or intradermal injection.

The dose of proteins of the invention to be administered can be readily determined by those skilled in the art, based on the usual patient symptoms discussed above. The dosage of proteins of the invention to be given in a bolus injection is preferred to be between 20 ng and 300 mg. The bolus injection may be repeated over several days, or the proteins of the invention can be continuously infused. If given as an intravenous infusion, the amount of proteins of the invention to be infused over a 24-hour period is about 1 mg to about 100 mg.

The amount of proteins of the invention to administer may also be determined by maintaining the local tissue concentration of TGF-beta at a subnormal level, or about 1-1.000 mu g/ml..

Preferably, proteins of the invention is applied topically, injected at the site of the problem or injected intravenously. Most preferably, proteins of the invention is administered by bolus injection at the site where TGF-beta is to be controlled. By intravenous injection, proteins of the invention should be administered at a rate to maintain a circulating serum concentration sufficient to reduce the TGF-beta excess. Preferably, the patient is started with a relatively low dose of proteins of the invention. The low dose preferably should be continued until the patient's acute phase is ameliorated or adequately improved, as indicated appropriate physical findings and laboratory results. Such improvement may be evident in two to three weeks. In the absence of significant improvement, the dose of proteins of the invention should be increased.

Gene therapy methods of administering the molecules of the invention are also encompassed within the scope of the invention. The term "transformation" or "transform" refers to any genetic modification of cells and includes both "transfection" and "transduction". As used herein, "transfection of cells" refers to the acquisition by a cell of new genetic material by incorporation of added DNA. Thus, transfection refers to the insertion of nucleic acid (e.g., DNA) into a cell using physical or chemical methods. Several transfection techniques are known to those of ordinary skill. In contrast, "transduction of cells" refers to the process of transferring nucleic acid into a cell using a DNA or RNA virus. One or more isolated polynucleotide sequences encoding one or more TGF-beta RII:Fc proteins contained within the virus may be incorporated into the chromosome of the transduced cell. Alternatively, a cell is transduced with a virus but the cell will not have the isolated polynucleotide incorporated into its chromosomes but will be capable of expressing interferon extrachromosomally within the cell. According to one embodiment, the cells are transformed (i.e., genetically modified) ex vivo. The cells are isolated from a mammal (preferably a human) and transformed (i.e., transduced or transfected in vitro) with a vector containing an isolated polynucleotide such as a recombinant TGF-beta RII:Fc nucleotide operatively linked to one or more expression control sequences for expressing a recombinant secreted protein. The cells are then administered to a mammalian recipient for delivery of the protein in situ. Preferably, the mammalian recipient is a human and the cells to be modified are autologous cells, i.e., the cells are isolated from the mammalian recipient. The isolation and culture of cells in vitro has been reported. According to another embodiment, the cells are transformed or otherwise genetically modified in vivo. The cells from the mammalian recipient (preferably a human), are transformed (i.e., transduced or transfected) in vivo with a vector containing the similar isolated polynucleotide and the protein is delivered in situ.

The isolated polynucleotides encoding the fusion protein are introduced into the cell ex vivo or in vivo by genetic transfer methods, such as transfection or transduction, to provide a genetically modified cell. Various expression vectors (i.e., vehicles for facilitating delivery of the isolated polynucleotide into a target cell) are known to one of ordinary skill in the art. Typically, the introduced material includes an isolated polynucleotide of the invention together with a promoter to control transcription. If delivery of the TGF-beta RII:Fc polynucleotide is to specific tissues, it may be desirable to target the expression of this gene. For instance, there are many promoters described in the literature which are only expressed in certain tissues. Thus, by selecting the appropriate promoter (constitutive versus inducible; strong versus weak), it is possible to control both the existence and level of expression of a fusion protein in the genetically modified cell. If the gene encoding the fusion protein is under the control of an inducible promoter, delivery of the protein in situ is triggered by exposing the genetically modified cell in situ to conditions permitting transcription of the protein, e.g., by injection of specific inducers of the inducible promoters which control transcription of the agent. Recently, very sophisticated systems have been developed which allow precise regulation of gene expression by exogenously administered small molecules. These include, the FK506/Rapamycin system (Rivera et al., Nature Medicine 2(9): 1028-1032, 1996); the tetracycline system (Gossen et al., Science 268: 1766-1768, 1995), the ecdysone system (No et al., Proc. Nat. Acad. Sci., USA 93: 3346-3351,1996) and the progesterone system (Wang et al., Nature Biotechnology 15: 239-243,1997).

Accordingly, the amount of fusion protein that is delivered in situ is regulated by controlling such factors as: (1) the nature of the promoter used to direct transcription of the inserted polynucleotide (i.e., whether the promoter is constitutive or inducible, strong or weak or tissue specific); (2) the number of copies of the exogenous polynucleotide that are inserted into the cell; (3) the number of transduced/transfected cells that are administered (e.g., implanted) to the patient; (4) the size of an implant (e.g., graft or encapsulated expression system) in ex vivo methods; (5) the number of implants in ex vivo methods; (6) the number of cells transduced/transfected by in vivo administration; (7) the length of time the transduced/transfected cells or implants are left in place in both ex vivo and in vivo methods; and (8) the production rate of the fusion protein by the genetically modified cell.

Expression vectors compatible with mammalian host cells for use in gene therapy include, for example, plasmids; avian, murine and human retroviral vectors; adenovirus vectors; herpes viral vectors; parvoviruses; and non-replicative pox viruses. In particular, replication-defective recombinant viruses can be generated in packaging cell lines that produce only replication-defective viruses. See Current Protocols in Molecular Biology: Sections 9.10-9.14 (Ausubel et al., eds.), Greene Publishing Associcates, 1989. Specific viral vectors for use in gene transfer systems are now well established. See for example: Madzak et al., J. Gen . Virol., 73: 1533-36 (1992) (papovavirus SV40); Berkner et al., Curr. Top. Microbiol. Immunol., 158: 39-61 (1992) (adenovirus); Moss et al., Curr. Top. Microbiol. Immunol., 158: 25-38 (1992) (vaccinia virus); Muzyczka, Curr. Top. Microbiol. Immunol., 158: 97-123 (1992) (adeno-associated virus); Margulskee, Curr. Top. Microbiol. Immunol., 158: 67-93 (1992) (herpes simplex virus (HSV) and Epstein-Barr virus (HBV)); Miller, Curr. Top. Microbiol. Immunol., 158: 1-24 (1992) (retrovirus); Brandyopadhyay et al., Mol. Cell. Biol., 4: 749-754 (1984) (retrovirus); Miller et al., Nature, 357: 455-450 (1992) (retrovirus); Anderson, Science, 256: 808-813 (1992) (retrovirus). Preferred vectors are DNA viruses that include adenoviruses (preferably Ad-2 or Ad-5 based vectors), herpes viruses (preferably herpes simplex virus based vectors), and parvoviruses (preferably "defective" or non-autonomous parvovirus based vectors, more preferably adeno-associated virus based vectors, most preferably AAV-2 based vectors). See, e.g., Ali et al., Gene Therapy 1: 367-384,1994; U.S. Patent 4,797,368 and 5,399,346 and discussion below.

Adeno-associated viruses (AAV) have also been employed as vectors for somatic gene therapy. AAV is a small, single-stranded (ss) DNA virus with a simple genomic organization (4.7 kb) that makes it an ideal substrate for genetic engineering. Two open reading frames encode a series of *rep* and *cap* polypeptides. *Rep* polypeptides (*rep78, rep68, rep 62 and rep 40*) are involved in replication, rescue and integration of the AAV genome. The cap proteins (VP1, VP2 and VP3) form the virion capsid. Flanking the *rep* and *cap* open reading frames at the 5' and 3' ends are 145 bp inverted terminal repeats (ITRs), the first 125 bp of which are capable of forming Y- or T-shaped duplex structures. Of importance for the development of AAV vectors, the entire *rep* and *cap* domains can be excised and replaced with a therapeutic or reporter transgene. See B.J. Carter, in Handbook of Parvoviruses, ed., P. Tijsser, CRC Press, pp. 155-168 (1990). It has been shown that the ITRs represent the minimal sequence required for replication, rescue, packaging, and integration of the AAV genome. The AAV life cycle is biphasic, composed of both latent and lytic episodes. During a latent infection. AAV virions enter a cell as an encapsidated ssDNA, and shortly thereafter are delivered to the nucleus where the AAV DNA stably integrates into a host chromosome without the apparent need for host cell division. In the absence of a helper virus, the integrated AAV genome remains latent but capable of being activated and rescued. The lytic phase of the life cycle begins when a cell harboring an AAV provirus is challenged with a secondary infection by a herpesvirus or adenovirus which encodes helper functions that are required by AAV to aid in its excision from host chromatin (B.J. Carter, supra). The infecting parental single-stranded (ss) DNA is expanded to duplex replicating form (RF) DNAs in a *rep* dependent manner. The rescued AAV genomes are packaged into preformed protein capsids (icosahedral symmetry approximately 20 nm in diameter) and released as infectious virions that have packaged either + or - ssDNA genomes following cell lysis. AAV have significant potential in gene therapy. The viral particles are very stable and recombinant AAVs (rAAV) have "drug-like" characteristics in that rAAV can be purified by pelleting or by CsCl gradient banding. They are heat stable and can be lyophilized to a powder and rehydrated to full activity. Their DNA stably integrates into host chromosomes so expression is long-term. Their host range is broad and AAV causes no known disease so that the recombinant vectors are non-toxic. Recently, recombinant baculovirus, primarily derived from the baculovirus *Aurographa californica* multiple nuclear polyhedrosis virus (AcMNPV), has been shown to be capable of transducing mammalian cells in vitro. (See Hofmann, C., Sandig, V., Jennings, G., Rudolph, M., Schlag, P., and Strauss. M. (1995), "Efficient gene transfer into human hepatocytes by baculovirus vectors", Proc. Natl. Acad. Sci. USA 92. 10099-10103; Boyce, F.M. and Bucher, N.L.R. (1996) "Baculovirus-mediated gene transfer into mammalian cells", Proc. Natl. Acad. Sci. USA 93, 2348-2352). Recombinant baculovirus has several potential advantages for gene therapy. These include a very large DNA insert capacity, a lack of a pre-existing immune response in humans, lack of replication in mammals, lack of toxicity in mammals, lack of expression of viral genes in mammalian cells due to the insect- specificity of the baculovirus transcriptional promoters, and, potentially, a lack of a cytotoxic T lymphocyte response directed against these viral proteins

The invention will be illustrated by the following, non-limiting examples.

### Example 1: Construction and Expression of Soluble-Rabbit TGFβR:Fc Fusion Protein

A full length clone (MIS_3f11), encoding the rabbit Type II TGF-beta ("TGFβ) receptor, is isolated from a rabbit ovary cDNA library as described (di Clemente et al., Mol. Endocrinol. 8:1006 [1994]). The recombinant rabbit TGFβRII:Fc fusion gene, consisting of the extracellular domain of the rabbit type II receptor (Nucleotide 832-1311 from SEQ ID NO.: 2) fused to the Fc portion of human IgG1 (Nucleotide 1312-1995 from SEQ ID NO.: 2), is constructed as follows:
Fragment 1) a 479 bp fragment , containing the extracellular domain of the rabbit TGFβ type II receptor, is amplified from clone MIS_3f1 by conventional PCR using the appropriate oligonucleotide primers (SEQ ID NO.: 5 and SEQ ID NO.: 6). These oligonucleotides confer flanking Not 1 and Sal 1 restriction enzyme sites on the resulting PCR product, which is subsequently digested with these two restriction enzymes.
Fragment 2) pSAB 132 (Miller et al., J. Exp. Med. 178:211 [1993]) is digested with the restriction enzyme Not1, and the terminal phosphate groups are removed by digestion with calf alkaline intestinal phosphatase.
Fragment 3) pSAB144 is constructed. The human IgG1 heavy (H) chain constant region containing the hinge region, the C_{H}2 and the C_{H}3 sequences, are isolated with H chain-specific primers by PCR amplification of cDNA from the RNA of COS-7 cells that had been transfected with plasmids containing 3 kilobase pair genomic inserts (Miller et al., J. Exp. Med. 178:211 [1993]). Heavy chain specific oligonucleotides primers are designed so that the resulting C_{H}2 and C_{H}3IgG1 PCR amplified sequences were flanked by a 5' Sal1 and a 3' Not1 restriction enzyme recognition site. pSAB 144 is digested with the restriction enzymes Not1 and Sal1 to release a 700 base pair fragment containing the Fc portion of human IgG1.

Fragments 1,2, and 3 are ligated together, and transformed into transformation competent bacteria. Plasmids are recovered from the transformants and analyzed by restriction enzyme digestions and gel electrophoresis for the correct orientation of assembled fragments. This construct is comprises the recombinant rabbit TGFβRII:Fc fusion gene, and the entire coding sequence is confirmed by DNA sequencing (SEQ ID NO.: 1 and 2).

The recombinant rabbit TGFβRII:Fc fusion gene is transfected into Chinese hamster ovary (CHO) cells by electroporation at 280 volts. After the initial transfection, cells are selected for the expression dihydrofolate reductase (DHFR) by subculturing in selective medium lacking glycine, hypoxanthine, and thymidine. The resulting colonies are then transferred to 24 well plates and analyzed for rabbit TGFβR:Fc fusion gene expression. The highest expressing cultures are subjected to amplification by exposure to increasing amounts of methotrexate. Cells able to grow at 25 nM methotrexate are cloned by limiting dilution in 96 well plates. The highest expressing clones are transferred to suspension culture and the clone expressing the highest levels of TGFβRII:Fc is selected for production of TGFβRII:Fc.

Those proficient in the art can construct and express a similar recombinant gene containing the extracellular domain of the human TGFβ type II receptor by isolating the human type II TGFβ receptor gene from commercially available human ovary cDNA (Clontech) and substituting the oligonucleotide primer described in SEQ ID NO.: 7 for the oligonucleotide primer described in SEQ ID NO.: 6. The DNA sequence of the resulting human TGFβR:Fc fusion gene is described (SEQ ID NO.: 3 and SEQ ID NO. 4).

### Example 2: The TGFβR:Fc Fusion Protein binds TGFβ in vitro.

The recombinant rabbit TGFβR:Fc fusion gene (SEQ ID NO: 2 contained within expression vector SEQ ID NO.: 1) is transfected into COS cells by electroporation at 280 volts. Transfected cells are grown in DMEM, supplemented with 10% fetal bovine serum. After 5 days, the cells are removed from the culture by centrifugation and the supernatant is assessed for the expression of recombinant rabbit TGFβRII:Fc by SDS/Polyacylamide gel electrophoresis (SDS/PAGE). TGFβRII:Fc present in the cell culture supernatant is tested for the ability to bind TGFβ. [¹²⁵I]-TGFβ (NEN Life Science Products) is incubated for 2.5 hours with conditioned cell culture medium containing rabbit TGFβRII:Fc, or control IgG, and Protein A-Sepharose which binds to the Fc portion of IgG. The resulting Protein A: Fc complexes are recovered by centrifugation, washed in phosphate buffered saline, and analyzed by SDS/PAGE and autoradiography. TGFβRII:Fc, but not control IgG immunoprecipitates [¹²⁵I]-TGFβ.

### Example 3: Mink Lung Cell Proliferation Assay

The proliferation of mink lung epithelial cells (CCL64) is inhibited by TGFβ1. Thus, we can test the ability of TGFβR:Fc to block the anti-proliferative effect of TGFβ1. CCL64 cells are maintained in MEM supplemented with 100 units/ml penicillin, 100 µg/ml streptomycin, and 10% fetal bovine serum. To test, serial dilutions of TGFβR:Fc are incubated with 0.1 ng/ml TGFβ1 for 1 hr in assay medium (MEM supplemented with 100 units/ml penicillin, 100 µg/ml streptomycin, and 10% fetal bovine serum) in a 96-well microtiter tissue culture plate. CCL64 cells are resuspended in assay medium and added to the assay plate at a concentration of 4000 cells per well. The cells are incubated at 37 degrees C for 72 hours and pulsed with [3H] thymidine (70-86 Ci/mmol) for an additional 4 hours. DNA synthesis, which reflects cell proliferation, is determined by measuring incorporation of [3H] thymidine. When cell are allowed to proliferate in media alone, the amount of [3H] thymidine incorporated is measured as 188,745 counts per minute (CPM). When TGFβ1 is included in the media, proliferation is inhibited, and the amount of [3H] thymidine incorporated is measured as 49088 counts per minute. Below, the results are shown when increasing amounts of TGFβR:Fc or control IgG are added to the media:

| **Concen.** | **IGF**β**RII:** | **Control** |
|---|---|---|
| **µg/ml** | **Fc (CPM)** | **IgG** |
| | | **(CPM)** |
| 10 | 181933 | 53619 |
| 5 | 179461 | 55097 |
| 2.5 | 178770 | 54443 |
| 1.25 | 168020 | 51443 |
| 0.62 | 150106 | 56683 |
| 0.31 | 118613 | 52415 |
| 0.16 | 75112 | 52830 |
| 0.08 | 63841 | 61894 |
| 0.04 | 49929 | 54395 |
| 0.02 | 57685 | 42993 |

The amount of TGFβRII:Fc required to inhibit the anti-proliferative effect of 0.1 ng/ml TGFβ1 by 50% equals 0.5 µg/ml.

### Example 4: Rat Vessel Injury Model

Male Sprague-Dawley rats weighing 400g and about 3-4 months of age (Bantin & Kingman, Edwards, WA) were used throughout. All surgical procedures are carried out under general anesthesia by intraperitoneal injection of xylazine (2.2 mg/kg, AnaSed TM, Lloyd Laboratories, Shenandoah, IA) and ketamine (540 mg/kg, Ketaset TM, Aveco Co., Fort Dodge, IA). The left common carotid artery is denuded with a 2F balloon catheter by introducing the catheter through the external carotid artery. The distal left common carotid and external carotid arteries are exposed through a midline wound in the neck. The catheter is passed three times with the balloon distended sufficiently with saline to generate slight resistance; this method produces distension of the carotid itself, the external carotifd is ligated ater remoeval of the catherter and the wound colosed.

Experimental treatments include a series of 1 ml intravenous injection of TGF-beta Type II receptor:Fc (SEQ ID NO: 8) in PBS given every other day (post-operation) at 5 mg/kg. After 14 days post balloon catheter denudation, all rats are anesthetized and the carotid arteries fixed by perfusion at 120 mm Hg pressure with 1% paraformaldehyde and 1.25% glutaraldehyde in 0.1M phosphate buffer, pH 7.4 via a large cannula placed retrograde in the abdominal aorta. Ten minutes before fixation, these animals are given an intravenous injection of Evans blue (0.3 ml in 5% saline solution). After 5 minutes of perfusion, the entire left and right common carotid arteries are retrieved, includigin the aortic arch. The vessels are further fixed by immersion in the same fixative as was used for perfusion.,

Arterial segments are assayed for the presence or absence of endothelium by obtaining three segments from the denuded, blue-stained left carotid artery and emdedding them in paraffin for cross sectioning using a "Micron" microtome. For measuring intimal areas, photomicrographs are obtained from 3-4 sections from each animal. The photomicrographs are digitized and anlaysed with image analyusis software (NIH Image 1.55 for MacIntosh). Intimal areas are measured by determining the area between liumen and internal elastic lamina. Medial areas are determined by measuring the area beween internal and external elastic lamina. Intimal/medial area ratios are calculated form the measurements.
Effect of TGF-beta RII:Fc (SEQ ID NO: 8) on Re-Stenosis in Rat Model

**Table 1: Intimal area (mm2)**

| Treatment | Mean Area | Std. Dev | Std. Error. |
|---|---|---|---|
| **Control (n=4)** | **0.15** | **0.015** | **0.008** |
| **Experimental:** | **0.068** | **0.016** | **0.007** |
| **5 mg/kg/dose** | | | |
| **35 mg/kg per course of therapy (n=5)** | | | |

An unpaired T-test between treatments resulted in a statistically significant p= 0.0001.

**Table 2: Intimal/Medial area ratios**

| Treatment | Mean Ratio | Std. Dev. | Std. Error |
|---|---|---|---|
| **Control (n=4)** | **0.706** | **0.03** | **0.015** |
| **Experimental:** | **0.360** | **0.137** | **0.061** |
| **5 mg/kg/dose** | | | |
| **35 mg/kg per course of therapy (n=5)** | | | |

An unpaired t-Test between treatments resulted in a statistically significant p= 0.0018, indicating that treatment with SEQ ID NO: 8 significantly reduced the formation of intima relative to the media as compared to non-trreated groups.

### Example 5: Lung Fibrosis Model

An excess accumulation of collagen in the lungs is a hallmark of lung fibrosis. The amount of collagen in tissue depends on the rate of collagen synthesis and collagen degradation and in cases of fibrosis, the rate of collagen synthesis predominates the rate of collagen degradation.

Chronic respiratory disease free Golden Syrain hamster weighing 120-130g are purchased from Charles River (Boston. MA) and housed in plastic cages in groups of 4 in facilities approved by the American Association for Accreditation of Laboratory Animal Care. The animals are allowed to acclimate for one week to laboratory conditions prior to starting the experiments. They have access to Rodent Laboratory Chow 5001 (Purina Mills, Inc., St. Louis, MO) and water *ad libitum* and housed in a room which gets the filtered air and has 12hr/12hr light/dark cycle. Hamsters will be assigned into the following groups for the biochemical and histopathological studies.

| Treatment Group | Biochemical Study (n) | Histopathology Study (n) |
|---|---|---|
| Saline IT + Saline IP | 10 | 6 |
| Bleomycin IT + saline IP | 10 | 6 |
| Bleomycin IT + TGF-beta Type II receptor:Fc | 10 | 6 |
| Saline IT + TGF-beta receptor Type II:Fc | 10 | 6 |

Bleomycin sulfate is dissolved in pyrogen free sterile isotonic saline just before intratraceheal (IT) instillation. Under pentobarbital anesthesia (25-35mg/kg ip) hamsters in appropriate group receive either bleomycin (5.5 units/kg/4ml) or an equivalent volume (4ml/kg) of pyrogen free isotonic saline through transoral route. The TGF-β Type II receptor:Fc is administered by intraperitoneal (IP) or intratrachial route at a therapeutic dose to hamster in appropriate groups twice a week for 21-28 days post installation. Thereafter, the animals in each group are killed by an overdose of sodium pentobarbital (100-125 mg/kg ip) and their lungs processed for biochemical and histopathological studies.

### 1. Biochemical Study

The lungs of animals used for biochemical studies are perfused *in situ* via the right ventricle with ice-cold isotonic saline to wash out blood from the pulmonary vasculature through an opening in the left ventricle. The lung lobes are quickly dissected free of non-parenchymal tissue, dropped in liquid nitrogen for quick freezing and then stored at -80C. The frozen lungs are later thawed and homogenized in 0.1 M KC, 0.02 M Tris buffer (pH 7.6) with a Polytron homogenizer. Several 1 ml aliquots of the homogenate are stored at -80C until the time various biochemical assays are carried out.

Hydroxyproline content of the lung homogenate as a measure of collagen content is quantitated by the techniques of Woessner, Arch. Biochem. Biophys. 93: 440-447 (1961) and lipid peroxidation by the method as described in Giri, et al.,, Biochem. Pharmacol. 54: 1205-1216 (1997).

### 2. Histopathological Study

Hamsters are anesthetized to a level of cardiac cessation. The thorax is opened, the heart tied off at its base and the trachea cannulated. The lungs and heart will be removed *en bloc,* weighed and placed in a normal saline bath and the lungs instilled with a 1% glutaraldehyde-paraformaldehyde fixative in 0.12M cacodylate buffer at 400m Osm at 30 cm H₂0 presure. The lungs are fixed via this pressure for about 2 hours and then stored in fixative with the tracheas occluded. Before embedding, the lung is isolated from the heart and all non-pulmonary tissue by blunt dissection and removed. Blocks of tissue are cut from at least two sagittal slabs (2-3mm thick) from the right cranial, right caudal, and left lung lobes of each lung. Each block is cut with about a 1 cm² face. The blocks are dehydrated in a graded series of ehtanol and embedded in paraffin. Sections (5µm thick) are cut from the paraffin blocks and stained with haematoxylin and eosin for histological evaluations.

### 3. Data Analysis and Interpretation

The values for different biochemical determinants in the lungs of control and treated animals are expresed per mg lung protein or DNA. However, it should be noted that the expression of the data based on per mg of lung protein or DNA will tend to introduce artificial lowering of the values in treated animals. This problem is attributed to the infiltration of leukocytes, presence eof residual plasma protein in the lungs even after perfusion, and proliferation of type II cells and fibroblasts in a long term study in the lungs of famster treated with bleomycin. In order to avoid an artificial lowering of the values, the data will ebe expressed on a per lung basis. See Starcher et al., Am. Rev. Resp. Dis., 117:299-305 (1973) and Witschi, Essays Toxicol., 6:125-191 (1975).

The data are analyzed in terms of average values with their standard deviations and standard errors of means. Student's t-test, chi-square distributions, correlation coefficient, analysis of variance (ANOVA) and multiple comparison test will be applied to judge the significance of differences between the control and treatment groups usign a computer based statistical package (SAS/STAT Guide, 6th Ed. Cary, N.C. pp. 183-260 (1985)).

### Example 6: Glomerulonephritis Model

The effect of proteins of the invention is assessed in a glomerulonephritis model. Experimental glomerulonephritis is induced in rats with a single injection of anti-glomerular basement membrane nephrotixin serum (NTS), derived in rabbits. The experimental lesion is acute mesangial proliferative glomerulonephritis and is characterized by expansion of the mesangial matrix and hypercellularity. The injured cells also express more TGF-beta 1 mRNA and TGF-beta 1, which in turn stimulates the synthesis of two proteoglycans, biglycan and decorin. Of particular intererst, the nephritis reproducibly progresses through glomerular and tubulointerstitial scarring, to end stage renal disease.

First, glomerulonephritis is induced in rats by an intravenous injection of NTS serum. Next, for six days, two groups of rats are treated with daily intravenous injections of saline (the negative control group) or proteins of the invention. On the tenth day, the animals are sacrificed and slides are made of the kidneys, which are stained with periodic acid-Schiff solution to emphasize the pathological changes. The negative control kidneys have full-blown glomerulonephritis with reddish-pink amorphous fibrous material filling most of the glomerulus. The positive control kidneys have a staining pattern which is similar to a normal glomerulus. The kidney which is treated with proteins of the invention also has a normal appearance, indicating that the proteins of the invention blocks the response due to the secretion of overproduction of TGF-beta.

The extent of glomerular injury can be quantitated by performing glomerular cell counts from 30 randomly selected glomeruli from normal animals and nephritic animals in each group. By day 10, there are more cells in glomeruli from NTS-treated rats. Kidney function is also assessed by measuring levels of albumin in urine and by quantitating creatinine clearance.

Another measure of the effect of proteins of the invention on the disease process is to quantitate the amount of extracellular matrix accumulation in the glomeruli. The degree of glomerular matrix expansion is determined as the percentage of each glomerulus occupied by the mesangial matrix according to the method of Raij et al. (1984) Kidney Int. 26: 137-43. The TGF-beta RII:Fc-treated kidneys are expected to have similar percentages of mesangial matrix to that in normal kidney, and significantly less mesangial matrix than in the negative control kidneys.

### Example 7: Arthritis Model

Arthritis is induced in pathogen-free female LEW rats (Harlan Sprague Dawley, Indianapolis, Ind.) weighing about 100 grams. Each receives a dose of cell wall fragments from Group A streptococci (SCW) (30 mu g rhamnose/gm bodyweight), injected intraperitoneally (ip) according to the technique described in Brandes et al. (1991) J. Clin. Invest. 87:1108. SCW-injected and control LEW rats are given an intraarticular (IA) injection in one of the hind ankles of one of the following:
1. proteins of the invention in 25 mu 1 PBS,
2. PBS only, or
3. an IgG control

Joints are clinically monitored by determining the amount of joint erythema, swelling and distortion on a scale of 0 (normal) to 4 (severe inflammation). Radiographs are taken and are evaluated for soft tissue swelling, joint space narrowing, bone erosions and deformity. Tissue specimens are obtained and prepared for histopathologic analysis as described in Brandes et al., ibid. Total RNA is isolated from excised synovial tissues according to the method of Allen et al. (1990) J. Exp. Med. 171:231.

Injection of SCW produces an acute inflammatory response which is clinically detectable within hours and maximal in 3-5 days. When TGF-beta Type II receptor:Fc is injected directly into a joint before ip administration of the SCW, inflammation at 24 hours is significantly below that observed in joints with the irrelevant antibody. At the peak of the acute response, inflammation of such treated joints remains far below that of joints with the irrelevant antibody. Even if joints are injected with TGF-beta Type II receptor:Fc when inflammation is well developed (day 13), the fusion still has a significant anti-inflammatory effect, when compared to irrelevant antibody. Because proteins of the invention also bind TGF-beta , they have a similarly beneficial effect when given early or late in the inflammatory process. Other well established experimental models of RA include collagen and adjuvant-induced arthritis. See, for example Janusz MJ et al., Inflamm Res. 46:503-508 (1997); Myers et al., Life Sci 61:1861-1878 (1997); and Kock et al., Clin Immunol Immunopathol 86: 199-208 (1998).

## Claims

1. A composition comprising a TGF-β receptor II (TGF-β R II) fusion protein comprising
(a) a polypeptide encoded by the polynucleotide shown in SEQ ID NO: 2;
(b) a polypeptide comprising the amino acid sequence of SEQ ID NO: 8; or
(c) a polypeptide comprising an amino acid sequence which is at least 98% identical to the amino acid sequence of SEQ ID NO:8,
and a constant region of an immunoglobulin, wherein said TGF-β R II fusion protein binds TGP-β.

2. A composition comprising a TGF-β receptor II (TGF-β R II) fusion protein comprising
(d) a polypeptide encoded by the polynucleotide shown in SEQ ID NO: 2;
(e) a polypeptide comprising the amino acid sequence of SEQ ID NO: 8; or
(f) a polypeptide comprising an amino acid sequence which is at least 98% identical to the amino acid sequence of SEQ ID NO:8,
and a constant region of an immunoglobulin, wherein said TGF-β R II fusion protein binds TGF-β for use as a medicament.

3. The composition of claim 2, wherein the constant region is from IgG1.

4. The composition of claim 2 or 3, wherein the constant region comprises the hinge, CH2 or CH3 portion of IgG1.

5. The composition of any one of claims 2 to 4, wherein said TGF-β R II fusion protein is a homodimer.

6. Use of a TGF-β R II fusion protein comprising
(a) a polypeptide encoded by the polynucleotide shown in SEQ ID NO: 2 or 4;
(b) a polypeptide comprising the amino acid sequence of SEQ ID NO. 8 or 9; or
(c) a polypeptide comprising an amino acid sequence which is at least 98% identical to the amino acid sequence of SEQ ID NO:8 or 9,
and a constant region of an immunoglobulin, wherein said TGF-β R II fusion protein binds TGF-β for the preparation of a pharmaceutical composition for the treatment of a subject suffering from a fibroproliferative disorder.

7. The use of claim 6, wherein the fibroproliferative disorder is arthritis, diabetic nephropathy, glomerulonephritis, proliferative vitreoretinopathy, myelofibrosis, or a collagen vascular disorder selected from the group consisting of systemic sclerosis, polymyositis, scleroderma, dermatomyositis, or systemic lupus erythematosus.

8. The use of claim 6 or 7, wherein the constant region is from IgG1.

9. The use of any one of claims 6 to 8, wherein the constant region comprises the hinge, CH2 or CH3 portion of IgG1.

10. The use of any one of claims 6 to 9, wherein said TGF-β R II fusion protein is a homodimer.

## Patentansprüche

1. Zusammensetzung, umfassend ein TGF-β Rezeptor II-Fusionsprotein (TGF-β R II), umfassend:
(a) ein Polypeptid, das von dem in SEQ ID NO: 2 gezeigten Polynucleotid codiert wird;
(b) ein Polypeptid, das die Aminosäuresequenz von SEQ ID NO: 8 umfasst; oder
(c) ein Polypeptid, das eine Aminosäuresequenz umfasst, die zu mindestens 98% identisch zu der Aminosäuresequenz von SEQ ID NO: 8 ist,
und eine konstante Region eines Immunglobulins, wobei das TGF-β R II-Fusionsprotein TGF-β bindet.

2. Zusammensetzung, umfassend ein TGF-β Rezeptor II-Fusionsprotein (TGF-β R II), umfassend:
(d) ein Polypeptid, das von dem in SEQ ID NO: 2 gezeigten Polynucleotid codiert wird;
(e) ein Polypeptid, das die Aminosäuresequenz von SEQ ID NO: 8 umfasst; oder
(f) ein Polypeptid, das eine Aminosäuresequenz umfasst, die zu mindestens 98% identisch zu der Aminosäuresequenz von SEQ ID NO: 8 ist,
und eine konstante Region eines Immunglobulins, wobei das TGF-β R II-Fusionsprotein TGF-β bindet, zur Verwendung als Arzneimittel.

3. Zusammensetzung nach Anspruch 2, wobei die konstante Region von IgG1 ist.

4. Zusammensetzung nach Anspruch 2 oder 3, wobei die konstante Region den Gelenk-, CH2- oder CH3-Teil von IgG1 umfasst.

5. Zusammensetzung nach einem der Ansprüche 2 bis 4, wobei das TGF-β R II-Fusionsprotein ein Homodimer ist.

6. Verwendung eines TGF-β R II-Fusionsproteins umfassend
(a) ein Polypeptid, das von dem in SEQ ID NO: 2 oder 4 gezeigten Polynucleotid codiert wird;
(b) ein Polypeptid, das die Aminosäuresequenz von SEQ ID NO: 8 oder 9 umfasst; oder
(c) ein Polypeptid, das eine Aminosäuresequenz umfasst, die zu mindestens 98% identisch zu der Aminosäuresequenz von SEQ ID NO: 8 oder 9 ist,
und einer konstanten Region eines Immunglobulins, wobei das TGF-β R II-Fusionsprotein TGF-β bindet, für die Herstellung eines Arzneimittels zur Behandlung eines Individuums, das an einer fibroproliferativen Störung leidet.

7. Verwendung nach Anspruch 6, wobei die fibroproliferative Störung Arthritis, diabetische Nephropathie, Glomerulonephritis, proliferative Vitreoretinopathie, Myelofibrose oder eine Kollagenopathie ist, ausgewählt aus der Gruppe bestehend aus systemischer Sklerose, Polymyositis, Sklerodermie, Dermatomyositis oder systemischem Lupus erythematodes.

8. Verwendung nach Anspruch 6 oder 7, wobei die konstante Region von IgG1 ist.

9. Verwendung nach einem der Ansprüche 6 bis 8, wobei die konstante Region den Gelenk-, CH2- oder CH3-Teil von IgG1 umfasst.

10. Verwendung nach einem der Ansprüche 6 bis 9, wobei das TGF-β R II-Fusionsprotein ein Homodimer ist.

## Revendications

1. Composition comprenant une protéine de fusion du récepteur de type II du TGF-β (TGF-β R II) comprenant
(a) un polypeptide codé par le polynucléotide présenté dans SEQ ID N° : 2 ;
(b) un polypeptide comprenant la séquence d'acides aminés de SEQ ID N° : 8 ; ou
(c) un polypeptide comprenant une séquence d'acides aminés qui est identique à au moins 98 % à la séquence d'acides aminés de SEQ ID N° : 8
et une région constante d'une immunoglobuline, dans laquelle ladite protéine de fusion de TGF-β R II se lie au TGF-β.

2. Composition comprenant une protéine de fusion du récepteur de type II du TGF-β (TGF-β R II) comprenant
(d) un polypeptide codé par le polynucléotide présenté dans SEQ ID N° : 2 ;
(e) un polypeptide comprenant la séquence d'acides aminés de SEQ ID N° : 8 ; ou
(f) un polypeptide comprenant une séquence d'acides aminés qui est identique à au moins 98 % à la séquence d'acides aminés de SEQ ID N° : 8
et une région constante d'une immunoglobuline, dans laquelle ladite protéine de fusion de TGF-β R II se lie au TGF-β pour utilisation comme médicament.

3. Composition selon la revendication 2, dans laquelle la région constante provient d'une IgG1.

4. Composition selon la revendication 2 ou 3, dans laquelle la région constante comprend la portion charnière, la portion CH2 ou la portion CH3 d'une IgG1.

5. Composition selon l'une quelconque des revendications 2 à 4, dans laquelle ladite protéine de fusion de TGF-β R II est un homodimère.

6. Utilisation d'une protéine de fusion de TGF-β R II comprenant
(a) un polypeptide codé par le polynucléotide présenté dans SEQ ID N° : 2 ou 4 ;
(b) un polypeptide comprenant la séquence d'acides aminés de SEQ ID N° : 8 ou 9 ; ou
(c) un polypeptide comprenant une séquence d'acides aminés qui est identique à au moins 98 % à la séquence d'acides aminés de SEQ ID N° : 8 ou 9
et une région constante d'une immunoglobuline, dans laquelle ladite protéine de fusion de TGF-β R II se lie au TGF-β pour la préparation d'une composition pharmaceutique destinée au traitement d'un sujet souffrant d'un trouble fibroprolifératif.

7. Utilisation selon la revendication 6, dans laquelle le trouble fibroprolifératif est l'arthrite, la néphropathie diabétique, la glomérulonéphrite, la vitréorétinopathie proliférative, la myélofibrose, ou un trouble vasculaire du collagène choisi dans le groupe constitué par la sclérose systémique, la polymyosite, la sclérodermie, la dermatomyosite et le lupus érythémateux disséminé.

8. Utilisation selon la revendication 6 ou 7, dans laquelle la région constante provient d'une IgG1.

9. Utilisation selon l'une quelconque des revendications 6 à 8, dans laquelle la région constante comprend la la portion charnière, la portion CH2 ou la portion CH3 d'une IgG1.

10. Utilisation selon l'une quelconque des revendications 6 à 9, dans laquelle ladite protéine de fusion de TGF-β R II est un homodimère.
